# EUROPEAN PATENT APPLICATION

(11) **EP 4 792 828 A1**
(43) Date of publication of application: **19.08.2026**
(21) Application number: 24876564.6
(22) Date of filing: 09.10.2024
(51) Int. Cl.: A61K 38/26, A61K 9/08, A61K 47/10, C07K 14/605, A61P 3/10

(54) **GLP -1 PEPTIDE COMPOSITION AND USE THEREOF**

(30) Priority: 09.10.2023 CN 202311299125; 24.05.2024 CN 202410655994
(71) Applicant: GAN & LEE PHARMACEUTICALS CO., LTD., Beijing 101109 (CN)
(72) Inventor: WANG, Zaiyu, Beijing 101109 (CN); PENG, Yu, Beijing 101109 (CN); QU, Jia, Beijing 101109 (CN)
(74) Representative: Kraus & Lederer PartGmbB
(86) International application number: PCT/CN2024/123787
(87) International publication number: WO 2025/077751

(57) **Abstract**

Provided are a GLP-1 peptide pharmaceutical composition containing citric acid, a preparation and use therefor, and a kit comprising the composition and a use thereof. The pharmaceutical composition comprises GLP-1 peptide, a buffering agent, an osmotic pressure regulator, and/or an antioxidant, can enhance the stability of physicochemical and biological activities of GLP-1 peptide, and does not cause pain to a subject. Thus, a pharmaceutical composition suitable for clinical primary injection can be prepared. The pharmaceutical composition can effectively prevent the effective component GLP-1 peptide from losing efficacy due to various factors such as degradation and oxidation, thereby facilitating transportation, long-term storage and clinical use.

## Description

### Technical Field

The present invention relates to the field of biopharmaceuticals and relates to a stable pharmaceutical composition containing a GLP-1 peptide.

### Background Art

Glucagon-like peptide-1 (GLP-1) and its analogs and derivatives are highly effective in treating type 1 and type 2 diabetes. However, GLP-1 peptides tend to exhibit poor stability in liquid solutions, such as chemical instability. Therefore, there is a need to optimize formulation systems to provide a liquid pharmaceutical composition of a GLP-1 peptide with good stability.

### Summary

The present invention provides a liquid pharmaceutical composition of a GLP-1 peptide having superior chemical stability. In some embodiments, the present invention relates to a kit comprising the pharmaceutical composition as defined herein. In some embodiments, the present invention relates to the pharmaceutical composition as defined herein for medical use.

In some embodiments of the present invention, a GLP-1 peptide formulation that is stable during storage and delivery is provided. A stable formulation is one in which the GLP-1 peptide substantially retains its physical and chemical stability during storage, produces fewer impurities and generates fewer high molecular weight proteins under the desired storage conditions, and meets the storage and delivery requirements of injectable formulations. The major factors determining the shelf life are typically the formation of byproducts and degradation products. The formulations of the present invention achieve these desired levels of stability.

In addition to sufficient physical and chemical stability, the formulation should also possess an acceptable pH value and osmotic pressure for use. In particular, high concentrations of the active pharmaceutical ingredients can increase aggregation of the formulation. In some embodiments of the present invention, the pharmaceutical formulation can reduce GLP peptide aggregation within a specific concentration range, providing a stable pharmaceutical formulation for GLP-1 peptides from low to high concentrations.

In addition, in some embodiments of the invention, the concentration of the formulation components have been optimized from the perspective of patient injection comfort, providing a solution that alleviates pain for the patient.

In its broadest aspect, the present invention provides a pharmaceutical formulation (the formulation of the present invention) comprising a GLP-1 peptide as an active ingredient and a buffer system, an osmotic pressure regulator, and an antioxidant. The formulation of the present invention is a liquid (e.g., an aqueous solution). In some embodiments of the present invention, a stable GLP-1 peptide formulation suitable for subcutaneous administration is provided.

Specifically, the first aspect of the present invention provides a liquid pharmaceutical composition comprising:
a. a GLP-1 peptide or a pharmaceutically acceptable salt thereof;
b. an osmotic pressure regulator;
c. an antioxidant; and
d. a buffer;
   wherein, the GLP-1 peptide is selected from the following compounds:
   *N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Gly8, Arg34]GLP-1-(7-37) peptide,
   *N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
   *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide,
   *N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide,
   *N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Gly8, Arg34]GLP-1-(7-37) peptide,
   *N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide,
   *N*-ε²⁶-(23-carboxytricosanoylamino)-4(*S*)-carboxybutanoyl-[Gly8, Arg34]GLP-1-(7-37) peptide,
   *N*-ε²⁶-(19-carboxynonadecanoylamino)-4(*S*)-carboxybutanoyl-[Gly8, Arg34]GLP-1-(7-37) peptide,
   *N*-ε²⁶-(21-carboxyheneicosanoylamino)-4(*S*)-carboxybutanoyl-[Gly8, Arg34]GLP-1-(7-37) peptide,
   *N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Arg34]GLP-1-(7-37) peptide,
   *N*-ε²⁶-[2-(2-[2-(4-[19-carboxynonadecanoylamino]-4(*S*)-carboxybutanoylamino)ethoxy]ethoxy)acetyl][Arg34]GLP-1-(7-37) peptide,
   *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Arg34]GLP-1-(7-37) peptide,
   *N*-ε²⁶-[2-(2-[2-(4-[21-carboxyheneicosanoylamino]-4(*S*)-carboxybutanoylamino)ethoxy]ethoxy)acetyl][Arg34]GLP-1-(7-37) peptide,
   *N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Arg34]GLP-1-(7-37) peptide,
   *N*-ε²⁶-[2-(2-[2-(4-[23-carboxytricosanoylamino]-4(*S*)-carboxybutanoylamino)ethoxy]ethoxy)acetyl][Arg34]GLP-1-(7-37) peptide,
   *N*-ε²⁶-(23-carboxytricosanoylamino)-4(*S*)-carboxybutanoyl-[Arg34]GLP-1-(7-37) peptide,
   *N*-ε²⁶-(19-carboxynonadecanoylamino)-4(*S*)-carboxybutanoyl-[Arg34]GLP-1-(7-37) peptide,
   *N*-ε²⁶-(21-carboxyheneicosanoylamino)-4(*S*)-carboxybutanoyl-[Arg34]GLP-1-(7-37) peptide,
   *N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Gly8, Arg34]GLP-1-(7-37) peptide,
   *N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
   *N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Gly8, Arg34]GLP-1-(7-37) peptide,
   *N*-ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
   *N*-ε²⁶-(20-carboxyeicosanoylamino)-4(*S*)-carboxybutanoyl-[Gly8, Arg34]GLP-1-(7-37) peptide,
   *N*-ε²⁶-(22-carboxydocosanoylamino)-4(*S*)-carboxybutanoyl-[Gly8, Arg34]GLP-1-(7-37) peptide,
   *N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Arg34]GLP-1-(7-37) peptide,
   *N*-ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
   *N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Arg34]GLP-1-(7-37) peptide,
   *N*-ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
   *N*-ε²⁶-(20-carboxyeicosanoylamino)-4(*S*)-carboxybutanoyl-[Arg34]GLP-1-(7-37) peptide, and
   *N*-ε²⁶-(22-carboxydocosanoylamino)-4(*S*)-carboxybutanoyl-[Arg34]GLP-1-(7-37) peptide;
   preferably, the GLP-1 peptide is selected from the following compound:
      *N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Gly8, Arg34]GLP-1-(7-37) peptide,
      *N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
      *N*-ε²⁶-(19-carboxynonadecanoylamino)-4(*S*)-carboxybutanoyl-[Gly8, Arg34]GLP-1-(7-37) peptide,
      *N*-ε²⁶-(19-carboxynonadecanoylamino)-4(*S*)-carboxybutanoyl-[Arg34]GLP-1-(7-37) peptide,
      *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide, and
      *N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide.
      preferably, the GLP-1 peptide is the following compounds: 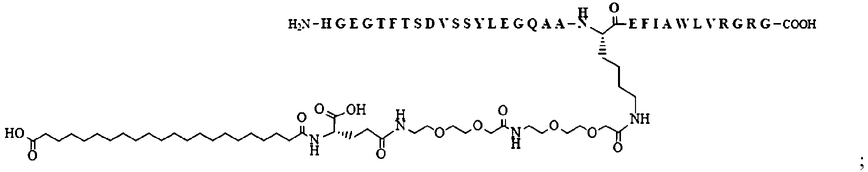 or

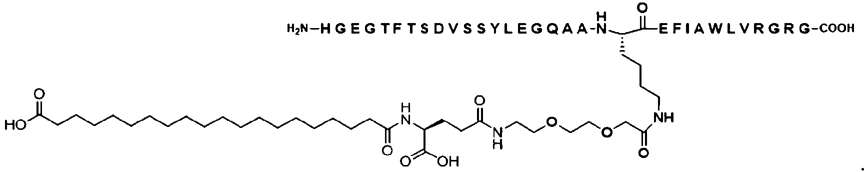

In some embodiments, the pharmaceutical composition comprises or does not comprise a preservative, preferably the pharmaceutical composition does not comprise a preservative, preferably the preservative is phenol or m-cresol, preferably the pharmaceutical composition does not comprise phenol or m-cresol.

In some embodiments, the buffer is at least one selected from the group consisting of an acetic acid-sodium acetate buffer, acetic acid-potassium acetate buffer, acetic acid-ammonium acetate buffer, and a phosphate buffer, and preferably a phosphate buffer; preferably the phosphate buffer is Na₂HPO₄.

In some embodiments, the osmotic pressure regulator is NaCl or propylene glycol.

In some embodiments, the antioxidant is at least one selected from the group consisting of ascorbic acid, methionine, citric acid and tartaric acid; preferably, the antioxidant is selected from citric acid and methionine; preferably, the citric acid is citric acid monohydrate.

In some embodiments, the pharmaceutical composition comprises more than about 0.5 mg/ml Na₂HPO₄, preferably about 0.5-20 mg/ml Na₂HPO₄, preferably about 1-20 mg/ml Na₂HPO₄, preferably about 1-10 mg/ml Na₂HPO₄, more preferably about 1-5 mg/ml Na₂HPO₄, and further preferably about 1.40 mg/ml, about 1.41 mg/ml, about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml Na₂HPO₄.

In some embodiments, the concentration of NaCl is more than about 0.5 mg/ml, preferably more than about 1 mg/ml, preferably about 1-30 mg/ml, preferably about 3-25 mg/ml, preferably about 5-15 mg/ml, preferably about 5-10 mg/ml, preferably about 5 mg/ml, about 5.5 mg/ml, about 6 mg/ml, about 7 mg/ml, about 8 mg/ml, about 8.10 mg/ml, about 8.15 mg/ml, about 8.20 mg/ml, about 8.25 mg/ml, about 8.30 mg/ml, about 9 mg/ml or about 10 mg/ml.

In some embodiments, the concentration of propylene glycol is more than about 1 mg/ml, preferably more than about 2 mg/ml, preferably about 5-25 mg/ml, preferably about 10-20 mg/ml, preferably about 12-18 mg/ml, preferably about 13 mg/ml, about 13.5 mg/ml, about 14 mg/ml, about 14.5 mg/ml, about 15 mg/ml, about 16 mg/ml or about 17 mg/ml.

In some embodiments, the concentration of the antioxidant is more than about 0.5 mM, preferably more than about 1 mM, preferably about 1-25 mM, preferably about 1-20 mM, preferably about 1-15 mM, preferably about 1-10 mM, preferably about 1 mM, about 2 mM, about 3 mM, about 4 mM, about 5 mM, about 6 mM, about 7 mM, about 8 mM or about 9 mM.

In some embodiments, the antioxidant is selected from citric acid at a concentration of about 1-25 mM, preferably at a concentration of about 1-20 mM, preferably at a concentration of about 1-15 mM, preferably at a concentration of about 1-10 mM, preferably at a concentration of about 1 mM, about 2 mM, about 3 mM, about 4 mM, about 5 mM, about 6 mM, about 7 mM, about 8 mM or about 9 mM; preferably the citric acid is citric acid monohydrate.

In some embodiments, the concentration of the GLP-1 peptide is at least about 1 mg/ml, preferably at least about 2 mg/ml, preferably about 1-80 mg/ml, preferably about 1-70 mg/ml, preferably about 1-65 mg/ml, preferably about 1-60 mg/ml, preferably about 2-60 mg/ml, preferably about 2-50 mg/ml, more preferably about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml, about 28 mg/ml, about 29 mg/ml, about 30 mg/ml, about 31 mg/ml, about 32 mg/ml, about 33 mg/ml, about 34 mg/ml, about 35 mg/ml, about 36 mg/ml, about 37 mg/ml, about 38 mg/ml, about 39 mg/ml, about 40 mg/ml, about 41 mg/ml, about 42 mg/ml, about 43 mg/ml, about 44 mg/ml, about 45 mg/ml, about 46 mg/ml, about 47 mg/ml, about 48 mg/ml, about 49 mg/ml,or about 60mg/ml, more preferably about 2mg/ml, about 3mg/ml, about 5mg/ml, about 6mg/ml, about 12mg/ml, about 15mg/ml, about 18mg/ml, about 24mg/ml, about 30mg/ml, about 36mg/ml, or about 48mg/ml; further preferably about 3mg/ml, about 6mg/ml, about 12mg/ml, about 18mg/ml, about 24mg/ml, about 36mg/ml, or about 48mg/ml.

In some embodiments, the pH of the pharmaceutical composition is about 6.5 to about 8.5, more preferably about 7.0 to about 8.5, further preferably about 7.1 to about 8.3, further preferably about 7.2, about 7.3, about 7.4, about 7.5, about 7.6, about 7.7, about 7.8, about 7.9, about 8.0, about 8.1, or about 8.2.

Another embodiment of the present invention also provides a liquid pharmaceutical composition comprising:
about 1-60 mg/ml, preferably about 1-70 mg/ml, preferably about 1-65 mg/ml, preferably about 1-60 mg/ml, preferably about 2-60 mg/ml, preferably about 2-50 mg/ml, more preferably about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml, about 28 mg/ml, about 29 mg/ml, about 30 mg/ml, about 31 mg/ml, about 32 mg/ml, about 33 mg/ml ml, about 34 mg/ml, about 35 mg/ml, about 36 mg/ml, about 37 mg/ml, about 38 mg/ml, about 39 mg/ml, about 40 mg/ml, about 41 mg/ml, about 42 mg/ml, about 43 mg/ml, about 44 mg/ml, about 45 mg/ml, about 46 mg/ml, about 47 mg/ml, about 48 mg/ml, about 49 mg/ml, further preferably about 2 mg/ml, about 3 mg/ml, about 5 mg/ml, about 6 mg/ml, about 12 mg/ml, about 15 mg/ml, about 18 mg/ml, about 24 mg/ml, about 30 mg/ml, about 36 mg/ml, or about 48 mg/ml; further preferably about 3 mg/ml, about 6 mg/ml, about 12 mg/ml, about 18 mg/ml, about 24 mg/ml, about 36 mg/ml, or about 48 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 1-30 mg/ml, preferably about 3-25 mg/ml, preferably about 5-15 mg/ml, preferably about 5 mg/ml, about 6 mg/ml, about 7 mg/ml, about 8 mg/ml, about 8.10 mg/ml, about 8.15 mg/ml, about 8.20 mg/ml, about 8.25 mg/ml, about 8.30 mg/ml, about 8.50 mg/ml, about 9 mg/ml or about 10 mg/ml of NaCl;
about 1-25 mM, preferably about 1-20 mM, more preferably about 1-15 mM, further preferably about 1-10 mM, further preferably about 1 mM, about 2 mM, about 3 mM, about 4 mM, about 5 mM, about 6 mM, about 7 mM, about 8 mM or about 9 mM citric acid;
about 1-20 mg/ml, preferably about 1-10 mg/ml, more preferably about 1-5 mg/ml, and further preferably about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of Na₂HPO4; and
the pH of the pharmaceutical composition is about 6.5 to about 8.5, more preferably about 7.0 to about 8.5, further preferably about 7.1 to about 8.3, further preferably about 7.2, about 7.3, about 7.4, about 7.5, about 7.6, about 7.7, about 7.8, about 7.9, about 8.0, about 8.1, or about 8.2.

Another embodiment of the present invention also provides a liquid pharmaceutical composition comprising:
about 1-60 mg/ml, preferably about 1-70 mg/ml, preferably about 1-65 mg/ml, preferably about 1-60 mg/ml, preferably about 2-60 mg/ml, preferably about 2-50 mg/ml, more preferably about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13mg/ml, about 14mg/ml, about 15mg/ml, about 16mg/ml, about 17mg/ml, about 18mg/ml, about 19mg/ml, about 20mg/ml, about 21mg/ml, about 22mg/ml, about 23mg/ml, about 24mg/ml, about 25mg/ml, about 26mg/ml, about 27mg/ml, about 28mg/ml, about 29mg/ml, about 30mg/ml, about 31mg/ml, about 32 mg/ml, about 33mg/ml, about 34mg/ml, about 35mg/ml, about 36mg/ml, about 37mg/ml, about 38mg/ml, about 39mg/ml, about 40mg/ml, about 41mg/ml, about 42mg/ml, about 43mg/ml, about 44mg/ml, about 45mg/ml, about 46mg/ml, about 47mg/ml, about 48mg/ml, or about 49mg/ml, further preferably about 2 mg/ml, about 3 mg/ml, about 5 mg/ml, about 6 mg/ml, about 12 mg/ml, about 15 mg/ml, about 18 mg/ml, about 24 mg/ml, about 30 mg/ml, about 36 mg/ml, or about 48 mg/ml; further preferably about 3 mg/ml, about 6 mg/ml, about 12 mg/ml, about 18 mg/ml, about 24 mg/ml, about 36 mg/ml, or about 48 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 5-25 mg/ml, preferably about 10-20 mg/ml, preferably about 12-18 mg/ml, preferably about 13 mg/ml, about 13.5 mg/ml, about 14 mg/ml, about 14.5 mg/ml, about 15 mg/ml, 16 mg/ml or about 17 mg/ml of propylene glycol;
about 1-25 mM, preferably about 1-20 mM, more preferably about 1-15 mM, further preferably about 1-10 mM, further preferably about 1 mM, about 2 mM, about 3 mM, about 4 mM, about 5 mM, about 6 mM, about 7 mM, about 8 mM or about 9 mM citric acid;
about 1-20 mg/ml, preferably about 1-10 mg/ml, more preferably about 1-5 mg/ml, and further preferably about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of Na₂HPO₄; and
the pH of the pharmaceutical composition is about 6.5 to about 8.5, more preferably about 7.0 to about 8.5, further preferably about 7.1 to about 8.3, further preferably about 7.2, about 7.3, about 7.4, about 7.5, about 7.6, about 7.7, about 7.8, about 7.9, about 8.0, about 8.1, or about 8.2.

Another embodiment of the present invention also provides a liquid pharmaceutical composition comprising:
about 3 mg/ml, about 6 mg/ml, about 12 mg/ml, about 18 mg/ml, about 24 mg/ml, about 30 mg/ml, about 36 mg/ml, about 48 mg/ml or about 60 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N-*ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 7 mg/ml, about 8 mg/ml, about 8.10 mg/ml, about 8.15 mg/ml, about 8.20 mg/ml, about 8.25 mg/ml, about 8.30 mg/ml, about 8.50 mg/ml, about 9 mg/ml, or about 10 mg/ml of NaCl;
about 1 mM, about 2 mM, about 3 mM, about 4 mM, about 5 mM, about 6 mM, about 7 mM, about 8 mM, 9 mM or about 10 mM citric acid;
about 1.42 mg/ml of Na₂HPO₄; and
the pH of the pharmaceutical composition is about 7.3.

Another embodiment of the present invention also provides a liquid pharmaceutical composition comprising:
about 3 mg/ml, about 6 mg/ml, about 12 mg/ml, about 18 mg/ml, about 24 mg/ml, about 30 mg/ml, about 36 mg/ml, or about 48 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N-*ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 14 mg/ml of propylene glycol;
about 1 mM, 2 mM, about 3 mM, about 4 mM, about 5 mM, about 6 mM, about 7 mM, about 8 mM, 9 mM or about 10 mM citric acid;
about 1.42 mg/ml of Na₂HPO₄; and
the pH of the pharmaceutical composition is about 7.3.

The second aspect of the present invention provides a pharmaceutical product comprising a container and the liquid pharmaceutical composition of the first aspect of the present invention placed in the container; preferably, the container is selected from a pen injection device, an automatic injection device, a syringe, and a vial.

In another embodiment of the present invention provides a pharmaceutical product comprising a container and a liquid pharmaceutical composition placed in the container, the container is deoxygenated and filled with nitrogen before the liquid pharmaceutical composition is placed in the container and/or during the process of the liquid pharmaceutical composition being placed in the container and/or after the liquid pharmaceutical composition is placed in the container,; the liquid pharmaceutical composition comprises:
about 3 mg/ml, about 6 mg/ml, about 12 mg/ml, about 18 mg/ml, about 24 mg/ml, about 36 mg/ml, about 48 mg/ml, or about 60 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N-*ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 3 mg/ml, about 4 mg/ml, about 5 mg/ml, about 6 mg/ml, about 7 mg/ml, about 8 mg/ml, about 8.10 mg/ml, about 8.15 mg/ml, about 8.20 mg/ml, about 8.25 mg/ml, about 8.30 mg/ml, about 8.50 mg/ml, about 9 mg/ml, or about 10 mg/ml of NaCl;
about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of Na₂HPO₄; and
the pH of the pharmaceutical composition is about 7.0, about 7.1, about 7.2, about 7.3, about 7.4, about 7.6, or about 7.7.

In another embodiment of the present invention provides a pharmaceutical product comprising a container and a liquid pharmaceutical composition placed in the container, the container is deoxygenated and filled with nitrogen before the liquid pharmaceutical composition is placed in the container and/or during the process of the liquid pharmaceutical composition being placed in the container and/or after the liquid pharmaceutical composition is placed in the container; the liquid pharmaceutical composition comprises:
about 2-60 mg/ml, preferably about 3 mg/ml, about 6 mg/ml, about 12 mg/ml, about 18 mg/ml, about 24 mg/ml, about 36 mg/ml, or about 48 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N-*ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 13 mg/ml, about 14 mg/ml, about 15 mg/ml, 16 mg/ml, or about 17 mg/ml of propylene glycol;
about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of Na₂HPO₄; and
the pH of the pharmaceutical composition is about 7.0, about 7.1, about 7.2, about 7.3, about 7.4, about 7.6, or about 7.7.

In some embodiments, the liquid pharmaceutical composition further comprises about 0.005 mM-25 mM, preferably about 0.01 mM-10 mM, further preferably about 0.1 mM-2.0 mM citric acid or methionine.

In some embodiments, deoxygenation of the container is achieved by filling the container with an inert gas; preferably, the inert gas is nitrogen.

In some embodiments, the container is a pen injection device, an automatic injection device, a syringe, or a vial.

The pharmaceutical composition according to the first aspect of the present invention, or the pharmaceutical product according to the second aspect of the present invention, wherein the pharmaceutical composition is for parenteral administration; preferably, the parenteral administration is subcutaneous administration.

A third aspect of the present invention provides a kit comprising a liquid pharmaceutical and instructions for use, wherein the liquid composition is as defined in the first aspect of the present invention.

Another embodiment of the present invention provides a kit comprising a liquid pharmaceutical composition and an injection device, wherein the liquid composition is as defined in the first aspect of the present invention, and the injection device is for administering the composition to a subject, wherein the injection device is selected from a durable pen and a prefilled pen injection device.

Use of the pharmaceutical composition of the first aspect of the present invention, or the pharmaceutical product of the second aspect of the present invention in the preparation of a medicament for treating diabetes, obesity, non-alcoholic fatty liver disease, Alzheimer's disease, or Parkinson's disease; preferably administering the pharmaceutical composition according to first aspect of the present invention to a subject in need thereof once a week, once every two weeks, or less frequently.

The pharmaceutical composition of the first aspect of the present invention, or the pharmaceutical product of the second aspect of the present invention, for use in treating diabetes, obesity, non-alcoholic fatty liver disease, Alzheimer's disease, or Parkinson's disease; preferably, the pharmaceutical composition according to first aspect of the present invention is administered to a subject in need thereof once a week, once every two weeks, or less frequently.

The fourth aspect of the present invention provides a method for treating diabetes, obesity, non-alcoholic fatty liver disease, Alzheimer's disease, or Parkinson's disease, the method comprising administering the pharmaceutical composition of the claim first aspect of the present invention, or the pharmaceutical product of the second aspect of the present invention, to a subject in need thereof; preferably, administering the pharmaceutical composition of the first aspect of the present invention, to a subject in need thereof once a week, once every two weeks, or less frequently.

The fifth aspect of the present invention provides a method for preparing the pharmaceutical composition of the first aspect, wherein the method comprises:
(1) dissolving a formulated amount of a buffer, an osmotic pressure regulator, and/or an antioxidant in water;
(2) dissolving a formulated amount of a GLP-1 peptide in the solution obtained in step (1), and adjusting the pH using sodium hydroxide and/or dilute hydrochloric acid;
(3) finally sterilizing the solution obtained in step (2) by filtering through a 0.22 µm sterile filter.

Another embodiment of the present invention provides a method for preparing the pharmaceutical composition according to the first aspect of the present invention, wherein the method comprises:
mixing the GLP-1 peptide, buffer, osmotic pressure regulator and antioxidant according to the formulation ;
preferably, the method further comprises:
   sterilizing the mixture obtained in step (2); preferably, the mixture obtained in step (2) is sterilized by filtering through a 0.22 µm sterile filter.

### Definitions

To better understand the present invention, definitions and explanations of relevant terms are provided below.

### GLP-1 peptide

The term "GLP-1 peptide" as used herein refers to "GLP-1 analogues" or "analogues of GLP-1," meaning peptides or compounds that are variants of human glucagon-like peptide-1 (GLP-1(7-37)), wherein one or more amino acid residues of GLP-1(7-37) are substituted, and/or one or more amino acid residues are deleted, and/or one or more amino acid residues are added. Specifically, the sequence of GLP-1(7-37) is shown as SEQ ID NO: 1 in the sequence listing. Peptides having the sequence shown in SEQ ID NO: 1 may also be referred to as "native" GLP-1 or "native" GLP-1(7-37).

In the sequence listing, the first amino acid residue (histidine) of SEQ ID NO:1 is numbered 1. However, in the following text, following established convention in the art, this histidine residue is numbered 7, and subsequent amino acid residues are numbered accordingly, concluding with glycine as number 37. Therefore, typically, the amino acid residue numbering or position numbering for the GLP-1(7-37) sequence discussed herein refers to the sequence starting with His at position 7 and ending with Gly at position 37.

[Gly8, Arg34] GLP-1-(7-37) peptide is a GLP-1 analog wherein Gly and Arg are substituted at positions 8 and 34, respectively, relative to GLP-1(7-37) (SEQ ID NO: 1). [Arg34] GLP-1-(7-37) peptide is a GLP-1 analog having Arg at position 34 corresponding to GLP-1(7-37) (SEQ ID NO: 1). Specifically, the amino acid sequence of the [Gly8, Arg34] GLP-1-(7-37) peptide is shown as SEQ ID NO: 2 in the sequence listing.

The GLP-1 peptide described herein may be prepared according to the method described in WO2021136303.

In the context of GLP-1 peptides or their analogs, the term "derivative" as used herein refers to a chemically modified GLP-1 peptide or analog wherein one or more substituents are covalently bonded to said peptides. Such substituents may also be referred to as side chains.

Unless otherwise specified, references to acylation of lysine residues should be understood as occurring at the ε-amino group.

The term "peptide", when used in reference to GLP-1 analogs such as those of the present invention, refers to a compound comprising a sequence of amino acids interconnected by amide (or peptide) bonds.

In one specific embodiment, the peptide is largely or primarily composed of amino acids interconnected via amide bonds (e.g., at least 50%, 60%, 70%, 80%, or at least 90% by molar mass). In another specific embodiment, the peptide is composed of amino acids interconnected via peptide bonds.

Amino acids are molecules containing both an amino group and a carboxyl group, optionally bearing one or more additional groups, commonly referred to as side chains.

The term "amino acid" comprises proteinogenic amino acids (encoded by the genetic code, including natural amino acids and standard amino acids), non-proteinogenic amino acids (not found in proteins, and/or not encoded in the standard genetic code), and synthetic amino acids. Non-proteinogenic amino acids are those that can be incorporated into peptides via peptide bonds but are not proteinogenic amino acids. Synthetic non-proteinogenic amino acids include those produced through chemical synthesis, namely D-isomers of amino acids encoded by the genetic code such as D-alanine and D-leucine, Aib (α-aminoisobutyric acid), Abu (α-aminobutyric acid), 3-aminomethylbenzoic acid, o-aminobenzoic acid, deamidated histidine, β-analogues of amino acids such as β-alanine, D-histidine, deamidated histidine, 2-aminohistidine, β-hydroxyhistidine, and homohistidine, etc.

Non-restrictive examples of amino acids not encoded by the genetic code include γ-carboxyglutamic acid, ornithine, D-alanine, D-glutamine, and phosphoserine. Non-limiting examples of synthetic amino acids include D-isomers of amino acids, such as D-alanine and D-leucine, Aib (α-aminoisobutyric acid), β-alanine, and des-aminohistidine (desH, also known as imidopropionic acid, abbreviated as Imp).

In the following text, all amino acids not explicitly labeled as optical isomers are understood to refer to the L-isomer (unless otherwise specified).

The term "stable" refers to the fact that all proteins within it essentially retain their physical, chemical, and biological activity after storage at the specified temperature range, such as 0-40°C. The antibody in the formulation does not maintain 100% physical, chemical, and biological activity after a certain storage period, yet the formulation may still be considered stable. If, after a specified storage period, the antibody retains approximately 90%, 91%, 92%, 93%, 94%, 95%, 96%, 97%, 98%, or more than 99% of its structure and function, the formulation can be deemed "stable".

The term "stable pharmaceutical composition" refers to a drug composition containing a GLP-1 peptide, such as a solution or suspension, wherein the composition contains at least 80% (w/v) of said GLP-1 peptide after storage (e.g., after 3 months of static storage at 25°C). Storage conditions for stability testing may be 2-8°C, such as 5°C, or at least 2.5 years at 5°C. Alternatively, storage conditions for stability testing may be at least 4 weeks, such as 6 weeks or 3 months, optionally at 30°C. The storage conditions for this stable pharmaceutical composition may be 1 or 2 years at 5°C. The storage conditions for this stable pharmaceutical composition may be 3 years at 5°C. Alternatively, the storage conditions may be 24 hours or 1 week at 25°C. In another alternative, the storage conditions may be at room temperature for up to two months.

In this application, when referring to specifically enumerated values or value ranges, the term "about" as used herein generally means within 20% of the given value or range, preferably within 10%, and more preferably within 5%.

The term "kit" may comprise both the pharmaceutical formulation described herein and a device used for administration, such as packaging the pharmaceutical formulation together with a device for administration, e.g., a syringe, inhaler, measuring cup, dropper, or applicator. The pharmaceutical formulation may be filled into the container defined above. The kit may optionally include instructions for use, which comprise intructions on dosage, dosing regimen, and route of administration.

The term "treatment" comprises therapeutic treatments, preventive treatments, and applications that reduce subjects' risk of developing diseases or other risk factors. Treatment includes, but not limited to, the complete cure of diseases, as well as the alleviation of symptoms or mitigation of underlying risks.

The term "subject" in this application may be used interchangeably with "patient" and refers to mammals, including but not limited to: humans and non-human primates, comprising apes and humans; mammalian equine animals (e.g., horses); mammalian farm animals (e.g., sheep, goats, etc.); mammalian pets (dogs, cats, etc.); and rodents (e.g., mice, rats, etc.).

The term "formulated amount" comprises the content of active substances or excipients in any embodiment of the present invention, as well as the content of active substances or excipients in all disclosed formulations.

The term "related substances" refers to impurities such as starting materials, intermediates, byproducts, and degradation products that may be introduced during drug manufacturing and storage. The presence of these impurities may affect the quality, safety, and efficacy of pharmaceutical products. In drug stability studies, related substances is a critical parameter for evaluation.

The term "high molecular weight proteins (HMWP) or high molecular weight protein" primarily refers to protein impurities with relatively high molecular weights. During the production and storage of pharmaceuticals, high molecular weight proteins may arise due to various factors such as aggregation or deformation. The detection and analysis of high molecular weight proteins constitute a critical step in ensuring the quality and safety of pharmaceutical products.

The term "inert gases" as used herein refers to noble gases or physiologically inert gases. Noble gases are elements in group 18 of the periodic table, while physiologically inert gases include nitrogen and methane.

### Detailed Description

The following detailed description of the embodiment of the present invention will be provided with reference to specific examples. However, those skilled in the art will understand that the following examples are provided merely to illustrate the invention and should not be construed as limiting the scope of the invention. Unless otherwise specified in the examples, standard conditions or manufacturer-recommended conditions shall be followed. Unless otherwise indicated, the reagents or instruments used for which the manufacturer is not specified are all conventional products that can be obtained through purchase on the market.

The active pharmaceutical ingredient GLP-1 peptide was formulated with various excipients using preparation methods known in the art.

Various prepared formulations were evaluated to determine the overall physicochemical stability of the active pharmaceutical ingredient GLP-1 peptide in the prepared formulations. The stability of the prepared formulations was analyzed using the following methods:
(1) Determination of related substances quantity

The content of impurities related to GLP-1 derivatives was determined by high-performance liquid chromatography (HPLC). The analysis was conducted on a Waters Kromasil 100-3.5-C8 column (4.6*250 mm) at a column temperature of 35°C and a sample cell temperature of 5°C, using an eluent flow rate of 1.0 mL/min. The elution was performed with a mobile phase composed of:
Phase A contained 90 mM potassium dihydrogen phosphate and 10% acetonitrile (v/v), pH 2.4.
Phase B was 75% (v/v) acetonitrile.
Gradient: Linear transition from 75%/25% A/B to 55%/45% A/B over 0-5 min, linear transition to 50%/50% A/B over 5-12 min, linear transition to 40%/60% A/B over 12-42 min, a linear change from 10%/90% A/B at 42-60 min, a linear change from 60-61 min to 75%/25% A/B, and an isometric gradient from 85%/15% A/B at 61-70 min.
The detection wavelength was 214 nm, the flow rate was 1.0 ml/min, and the injection volume was 15 µl. The increased related substances relative to day 0 was determined after storage at 4°C, 25°C, and 37°C for several days.

### (2) Determination of High Molecular Weight Proteins (HMWP)

High molecular weight proteins (HMWP) content was determined by high-performance liquid chromatography (HPLC) using a Waters TSKgel G2000SWXL (7.8*300 mm), 5 µm column. The column temperature was maintained at 30°C, and the sample cell temperature was set to 5°C. The mobile phase flow rate was 0.5 mL/min. The mobile phase consisted of 300 mL isopropanol, 400 mL glacial acetic acid, and 300 mL purified water. The detection wavelength was 276 nm, with a sample volume of 25 µl. After storage at 4°C, 25°C, and 37°C for several days, the increased HMWP relative to Day 0 was measured.

### Abbreviations

Na₂HPO₄ is disodium hydrogen phosphate;
NaOH is sodium hydroxide;
OEG is the amino acid residue -NH(CH₂)₂O(CH₂)₂OCH₂C(O)-;
OSu is succinimidyl-1-oxyl-2,5-dioxo-pyrrolidin-1-yloxy;
OtBu is oxy-tert-butyl;
HCl is hydrogen chloride;
γGlu or gGlu denotes γL-glutamoyl;
HPLC is High Performance Liquid Chromatography;
DCC is dicyclohexylcarbodiimide;
AEEA is 2-(2-(2-aminoethoxy) ethoxy)acetic acid.

### Example 1

### Preparation of the title compound: N-ε²⁶-[2-(2-[2-(2-[2-(2-[4-(21-carboxyheneicosanoylamino)-4(S)-carboxybutanoylamino] ethoxy) ethoxy] acetylamino) ethoxy] ethoxy) acetyl] [Gly8, Arg34] GLP-1-(7-37) peptide (Compound 1)

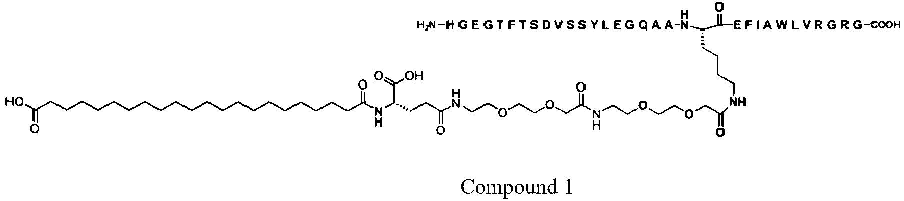

### 1. N-ε²⁶-[2-(2-[2-(2-[2-(2-[4-(21-carboxyheneicosanoylamino)-4(S)-carboxybutanoylamino] ethoxy) ethoxy] acetylamino) ethoxy] ethoxy) acetyl] [Gly8, Arg34] preparation of GLP-1-(7-37) peptide

The [Gly8, Arg34] GLP-1-(7-37) peptide was prepared using standard recombinant protein expression methods in this field (see Molecular Cloning: A Laboratory Manual (Fourth Edition), Michael R. Green, Cold Spring Harbor Press, 2012, for specific protocols). [Gly8, Arg34] GLP-1-(7-37) peptide (5 g, 1.48 mmol) was dissolved in 100 mM Na₂HPO₄ aqueous solution (150 mL), acetonitrile (100 mL) was added, and the pH was adjusted to 10-12.5 using 1 N NaOH. Tert-butyl eicosanedioate-γGlu (2xOEG-OSu)-OtBu (1.59 g, 1.63 mmol) was dissolved in acetonitrile (50 mL), and the [Gly8, Arg34] GLP-1-(7-37) peptide solution was slowly added. The pH was maintained between 10 and 12.5. After 120 minutes, the reaction mixture was added to water (150 mL), and the pH was adjusted to 5.0 using 1N HCl aqueous solution. The precipitate was separated by centrifugation and freeze-dried. The crude product was added to a mixture of trifluoroacetic acid (60 mL) and dichloromethane (60 mL), and stirred at room temperature for 30 minutes. The mixture was concentrated to approximately 30 mL, poured into ice-cold n-heptane (300 mL), and the precipitated product was separated by filtration, and washed twice with n-heptane. After the mixture was dried under vacuum, the product was purified by ion-exchange chromatography (Ressource Q, 0.25%-1.25% ammonium acetate gradient in 42.5% ethanol, pH 7.5) and was purified by reverse-phase chromatography (acetonitrile, water, TFA). Purified fractions were pooled, pH was adjusted to 5.2 with 1N HCl, and precipitate was separated and freeze-dried to afford the title compound.
LC-MS (Electrospray): m/z = 1035.8 [M+4H]⁴⁺

### 2. Preparation of the intermediate tert-butyl docosanedioyl-γGlu-(2xOEG-OSu)-OtBu

### 2.1 Tert-butyl docosanedioyl-OSu

Under a nitrogen protection, docosanedioic acid mono-tert-butyl ester(20 g, 50.17 mmol) and NHS (5.77 g, 50.17 mmol) were mixed in dichloromethane (400 mL). Triethylamine (13.95 mL) was added, and the resulting turbid mixture was stirred at room temperature. DCC (11.39 g, 55.19 mmol) was added and the mixture was stirred further overnight. The mixture was filtered, and the filtrate was concentrated to near dryness. The residue was combined with cold water and ethyl acetate, stirred for 20 minutes, and the layers were separated. The upper organic layer was washed with saturated brine. After the layers were separated, the upper organic layer was dried with anhydrous sodium sulfate, filtered, and the filtrate was concentrated under reduced pressure to near dryness. The mixture was dried under vacuum overnight to afford 24.12 g (97% yield) of tert-butyl docosanedioyl -OSu.

### 2.2 Tert-butyl docosanedioyl-γGlu-OtBu

Tert-butyl docosanedioyl-OSu (24.12 g, 48.66 mmol) was dissolved in dichloromethane (250 mL) and stirred. H-Glu-OtBu (10.88 g, 53.53 mmol), triethylamine (12.49 mL), and water (25 mL) were sequentially added. The mixture was heated to yield a clear solution, which was stirred at room temperature for 4 hours. Then 10% citric acid aqueous solution (200 mL) was added, and the layers were separated. The lower organic layer was washed with saturated saline solution. After the layers were separated, the lower organic layer was dried with anhydrous sodium sulfate, filtered, and the filtrate was concentrated under reduced pressure to near dryness. The mixture was dried under vacuum overnight to afford 27.27 g (96% yield) of tert-butyl docosanedioyl-γGlu-OtBu.

### 2.3 Tert-butyl docosanedioic-γGlu(OSu)-OtBu

Under a nitrogen atmosphere, tert-butyl docosanedioyl-γGlu-OtBu (27.27 g, 46.71 mmol) was dissolved in dichloromethane (300 mL), triethylamine (11.99 mL) was added, and the mixture was stirred for 10 min. NHS (5.38 g, 50.17 mmol) was then added, and DCC (10.60 g, 51.38 mmol) was then added. The mixture was stirred overnight at room temperature, filtered, and the filtrate was concentrated to near dryness. The residue was mixed with cold water and ethyl acetate, stirred for 20 minutes, and the layers were separated. The upper organic layer was washed with saturated brine. After the layers were separated, the upper organic layer was dried with anhydrous sodium sulfate, filtered, and the filtrate was concentrated under reduced pressure to near dryness. Methyl tert-butyl ether was added, stirred for 30 minutes, filtered by suction, and the filter cake was dried under vacuum overnight to afford 25.76 g (81% yield) of tert-butyl docosanedioyl-γGlu-(OSu)-OtBu.

### 2.4 Tert-butyl docosanedioic-γGlu-(2xOEG-OH)-OtBu

Tert-butyl docosanedioyl-γGlu-(OSu)-OtBu (25.76 g, 37.83 mmol) was dissolved in dichloromethane (250 mL) and the mixture was stirred. 2xAEEA (11.66 g, 37.83 mmol), triethylamine (9.71 mL), and water (25 mL) were added sequentially. The mixture was heated to afford a clear solution, which was stirred at room temperature for 4 hours. Then 10% citric acid aqueous solution (200 mL) was added, and the layers were separated. The lower organic layer was washed with saturated saline solution. After the layers were separated, the lower organic layer was dried with anhydrous sodium sulfate, filtered, and the filtrate was concentrated under reduced pressure to near dryness. The mixture was dried under vacuum overnight to afford 30.75 g (93% yield) of tert-butyl docosanedioyl-γGlu-(2xOEG-OH)-OtBu.

### 2.5 Tert-butyl docosanedioic-γGlu-(2xOEG-OSu)-OtBu.

Under a nitrogen atmosphere, tert-butyl docosanedioyl-γGlu-(2xOEG-OH)-OtBu (30.75 g, 35.18 mmol) was dissolved in dichloromethane (300 mL). Triethylamine (9.03 mL) was added and the mixture was stirred for 10 minutes, then the NHS (4.05 g, 35.18 mmol) was added. Subsequently, DCC (7.98 g, 38.70 mmol) was added. The mixture was stirred overnight at room temperature, filtered, and the filtrate was concentrated to near dryness. The residue was mixed with cold water and ethyl acetate, stirred for 20 minutes, and the layers were separated. The upper organic layer was washed with saturated brine. After the layers were separated, the upper organic layer was dried with anhydrous sodium sulfate, filtered, and the filtrate was concentrated under reduced pressure to near dryness. The mixture was dried under vacuum overnight to afford 31.09 g (91% yield) of tert-butyl docosanedioyl-γGlu-(2xOEG-OSu)-OtBu.

### Preparation of the title compound N-ε²⁶-[2-(2-[2-(4-[19-carboxynonadecanoylamino]-4(S)-carboxybutanoylamino)ethoxy]ethoxy)acetyl][Gly8, Arg34]GLP-1-(7-37) peptide (Compound 2)

*N-ε²⁶*-[2-(2-[2-(4-[19-carboxynonadecanoylamino]-4(S)-carboxybutanoylamino)ethoxy]ethoxy)acetyl][Gly8, Arg34]GLP-1-(7-37) peptide was prepared according to a procedure similar to that described in Part 1 of Example 1.
LC-MS (Electrospray): m/z = 992.52 [M+4H]⁴⁺

The intermediate tert-butyl-docosanedioyl-γGlu-(OEG-OSu)-OtBu was prepared by steps similar to those in Part 2 of Example 1.
LC-MS(Scie×100API):m/z=826.54(M+1)⁺

### Example 2: Effects of Antioxidants Citric Acid and Methionine on the Chemical Stability of GLP-1 Peptide Formulation

### 2.1 Preparation of pharmaceutical formulations

The compound 1 was dissolved in disodium hydrogen phosphate solution to the final concentrations specified in Table 1. Each auxiliary liquid was added sequentially according to the quantities listed in the table below, the pH was adjusted to the values indicated in the table, and the final formulation as shown in Table 1 was produced.

**Table 1: Formulation composition**

| Formulation No. | Compound 1 (mg/mL) | Na₂HPO₄ (mg/mL) | NaCl (mg/mL) | Phenol (mg/mL) | Propylene glycol (mg/mL) | Citric acid monohydrate (mM) | Methionine (mM) | pH |
|---|---|---|---|---|---|---|---|---|
| 2-1 | 10 | | - | 5.5 | 14 | - | - | |
| 2-2 | | | 8.25 | - | - | | | |
| 2-3 | | | | | | 5 | | |
| 2-4 | | | | | | - | 5 | |
| 2-5 | 20 | | | | | - | - | |
| 2-6 | | | | | | 5 | - | |
| 2-7 | | 1.42 | | | | - | 5 | 7.3 |
| 2-8 | 40 | | | | | | - | |
| 2-9 | | | | | | 5 | | |
| 2-10 | | | | | | - | 5 | |
| 2-11 | 60 | | - | 5.5 | 14 | - | - | |
| 2-12 | | | 8.25 | - | - | | | |
| 2-13 | | | | | | 5 | | |
| 2-14 | | | | | | - | 5 | |

### 2.2 Detection of related substances

The formulations 2-1 to 2-14 described above were filled into prefilled cartridges and subjected to stability studies at 4°C, 25°C, and 37°C. The increase in related substances relative to Day 0 for each formulation (formulations 2-1 to 2-14) over different days was measured. The test results are shown in Tables 2, 3, and 4, respectively.

**Table 2: Results of related substances for compound 1 formulations at 4°C**

| Formulation No. | Compound 1 (mg/mL) | Day0 | Day14 | Day28 | Day63 |
|---|---|---|---|---|---|
| 2-1 | 10 | 1.91 | 2.48 | 2.48 | 2.82 |
| 2-2 | | 1.86 | 2.41 | 2.48 | 2.67 |
| 2-3 | | 1.80 | 2.40 | 2.44 | 2.71 |
| 2-4 | | 1.92 | 2.50 | 2.43 | 2.77 |
| 2-5 | 20 | 1.83 | 2.41 | 2.52 | 2.77 |
| 2-6 | | 1.90 | 2.46 | 2.52 | 2.64 |
| 2-7 | | 1.81 | 2.47 | 2.54 | 2.78 |
| 2-8 | 40 | 1.89 | 2.44 | 2.59 | 2.84 |
| 2-9 | | 2.01 | 2.60 | 2.62 | 2.84 |
| 2-10 | | 1.95 | 2.55 | 2.53 | 2.84 |
| 2-11 | 60 | 1.53 | 1.79 | 1.84 | 2.09 |
| 2-12 | | 1.51 | 1.72 | 1.94 | 2.20 |
| 2-13 | | 1.57 | 1.65 | 1.90 | 2.23 |
| 2-14 | | 1.52 | 1.72 | 1.89 | 2.23 |

It can be seen from Table 2 that: the trends in related substances changes between compound 1 formulations containing antioxidants and the formulations containing preservatives were essentially consistent during long-term storage at 4°C. The overall increase in related substances for the high-concentration formulation of compound 1(60 mg/mL) was lower compared to the related substances at Day 0.

**Table 3: Results of related substances in compound 1 formulations under accelerated conditions at 25°C**

| Formulation No. | Compound 1 (mg/mL) | Day 0 | Day 7 | Day 14 | Day 21 | Day 28 | Day 35 | Day 43 | Day 49 | Day 56 | Day 63 | Day 91 |
|---|---|---|---|---|---|---|---|---|---|---|---|---|
| 2-1 | 10 | 1.91 | 2.07 | 2.85 | 2.91 | 3.09 | 3.20 | 3.43 | 3.27 | 3.43 | 3.90 | 4.21 |
| 2-2 | | 1.86 | 2.20 | 2.81 | 2.80 | 3.18 | 3.20 | 3.47 | 3.56 | 3.64 | 4.42 | 4.75 |
| 2-3 | | 1.80 | 2.48 | 2.87 | 2.82 | 3.02 | 3.94 | 3.71 | 3.39 | 4.50 | 3.94 | 4.21 |
| 2-5 | 20 | 1.83 | 2.26 | 2.79 | 2.85 | 3.25 | 3.19 | 3.86 | 3.55 | 3.92 | 4.58 | 5.51 |
| 2-6 | | 1.90 | 2.14 | 2.74 | 2.82 | 3.27 | 3.21 | 3.41 | 3.18 | 3.55 | 4.06 | 4.19 |
| 2-7 | | 1.81 | 2.22 | 2.79 | 2.79 | 3.10 | 3.23 | 3.36 | 3.29 | 3.60 | 3.82 | 4.35 |
| 2-8 | 40 | 1.89 | 2.23 | 2.79 | 2.90 | 3.36 | 3.45 | 3.73 | 3.72 | 3.99 | 4.53 | 5.38 |
| 2-9 | | 2.01 | 2.03 | 2.74 | 2.82 | 3.20 | 3.17 | 3.42 | 3.27 | 3.63 | 3.99 | 4.31 |
| 2-10 | | 1.95 | 2.19 | 2.67 | 2.95 | 3.34 | 3.32 | 3.45 | 3.53 | 3.70 | 4.36 | 4.85 |
| 2-11 | 60 | 1.53 | 1.76 | 1.93 | 2.08 | 2.38 | 2.40 | 2.65 | 2.60 | 2.90 | 3.89 | 3.67 |
| 2-12 | | 1.51 | 1.56 | 1.93 | 2.18 | 2.49 | 2.79 | 3.22 | 3.00 | 3.39 | 4.14 | 4.58 |
| 2-13 | | 1.57 | 1.70 | 1.91 | 2.12 | 2.46 | 2.53 | 2.99 | 2.84 | 3.10 | 3.90 | 3.66 |
| 2-14 | | 1.52 | 1.78 | 1.95 | 2.19 | 2.40 | 2.56 | 2.89 | 3.03 | 3.04 | 3.59 | 4.00 |

It can be seen from Table 3 that: the trend in related substances changes under accelerated conditions at 25°C was essentially consistent between compound 1 formulations containing antioxidants and the formulations containing preservatives. The difference at Day 91 was quite noticeable: In the compound 1 concentration groups of 10 mg/mL and 60 mg/mL, the trend in related substances after the addition of citric acid was essentially consistent with that of formulations containing preservatives. The increase in related substances relative to Day 0 was higher in groups without antioxidants. Notably, in the 20 mg/mL and 40 mg/mL groups, the increase in related substances relative to Day 0 was lower in the citric acid-containging group. Moreover, the increase in related substances relative to Day 0 was lower in the high-concentration compound 1 group (60 mg/mL). The addition of antioxidants slowed the increase in related substances content, with citric acid monohydrate demonstrating a more pronounced effect than methionine in mitigating this increase, thereby enhancing formulation stability.

**Table 4: Results of related substances in compound 1 formulations under accelerated conditions at 37°C**

| Formulation No. | Compound 1 (mg/mL) | Day 0 | Day 7 | Day 14 | Day 21 | Day 28 | Day 35 | Day 43 | Day 49 | Day 56 | Day 63 |
|---|---|---|---|---|---|---|---|---|---|---|---|
| 2-1 | 10 | 1.91 | 2.71 | 3.81 | 4.29 | 5.15 | 5.98 | 6.57 | 7.15 | 7.86 | 8.90 |
| 2-2 | | 1.86 | 3.01 | 4.12 | 5.32 | 6.96 | 7.99 | 8.93 | 9.76 | 10.94 | 12.85 |
| 2-3 | | 1.80 | 2.77 | 3.88 | 4.46 | 5.61 | 6.01 | 6.53 | 6.78 | 7.68 | 8.90 |
| 2-4 | | 1.92 | 2.92 | 3.80 | 4.49 | 5.39 | 6.54 | 7.04 | 7.47 | 8.62 | 10.13 |
| 2-5 | 20 | 1.83 | 2.90 | 4.29 | 5.60 | 7.20 | 8.16 | 8.84 | 9.89 | 10.31 | 12.60 |
| 2-6 | | 1.90 | 2.81 | 3.86 | 4.35 | 5.39 | 5.92 | 6.49 | 6.81 | 7.70 | 9.02 |
| 2-7 | | 1.81 | 2.92 | 3.69 | 4.89 | 5.94 | 6.94 | 7.50 | 8.69 | 9.90 | 10.69 |
| 2-8 | 40 | 1.89 | 3.13 | 4.27 | 5.45 | 6.61 | 7.20 | 7.82 | 8.08 | 9.00 | 10.00 |
| 2-9 | | 2.01 | 2.99 | 3.68 | 4.56 | 5.59 | 6.01 | 6.70 | 7.02 | 7.83 | 9.11 |
| 2-10 | | 1.95 | 3.04 | 3.84 | 4.98 | 6.30 | 6.99 | 7.25 | 8.05 | 8.88 | 9.79 |
| 2-11 | 60 | 1.53 | 2.53 | 2.95 | 3.80 | 4.64 | 5.13 | 5.94 | 6.43 | 7.33 | 8.48 |
| 2-12 | | 1.51 | 2.51 | 3.33 | 4.33 | 5.39 | 6.28 | 6.74 | 7.23 | 8.71 | 9.08 |
| 2-13 | | 1.57 | 2.47 | 3.10 | 3.78 | 4.73 | 5.55 | 5.86 | 6.49 | 7.32 | 8.42 |
| 2-14 | | 1.52 | 2.51 | 3.31 | 3.97 | 5.18 | 5.98 | 6.43 | 7.11 | 7.80 | 8.71 |

It can be seen from Table 4 that: formulations containing antioxidants and those containing preservatives exhibited essentially consistent trends in related substances changes during long-term accelerated storage at 37°C. A more pronounced difference was observed at Day 63: In the compound 1 concentration groups of 10 mg/mL and 60 mg/mL, the increase in related substances relative to Day 0 was higher in the group without antioxidants. In the 20 mg/mL and 40 mg/mL groups, the formulations containing citric acid show a lower increase in related substances relative to Day 0. The increase in related substances relative to Day 0 decreased with increasing concentration of compound 1 across all groups, meaning higher concentrations of compound 1 (e.g., 60mg/mL) resulted in lower related substances. Furthermore, formulations containing NaCl can slow the increase of related substances when antioxidants are added, compared to formulations containing propylene glycol, and citric acid monohydrate shows a more significant effect than methionine.

In summary, antioxidants, particularly citric acid monohydrate, can slow the increase of related substances in formulation compositions. Among these, citric acid monohydrate demonstrated a more pronounced slowing effect than methionine, thereby being more favorable for improving the stability of the formulations.

### 2.3 Detection of High Molecular Weight Proteins (HMWP)

Same to section 2.2, the increase in HMWP relative to Day 0 was measured at 4°C, 25°C, and 37°C for each formulation in Example 2. The results were shown in Tables 5, 6, and 7, respectively.

**Table 5: HMWP detection results for compound 1 formulations at 4°C**

| Formulation No. | Compound (mg/mL) | Day 0 | Day 14 | Day 28 | Day 63 |
|---|---|---|---|---|---|
| 2-1 | 10 | 0.17 | 0.19 | 0.25 | 0.26 |
| 2-3 | | 0.16 | 0.19 | 0.23 | 0.27 |
| 2-5 | 20 | 0.15 | 0.19 | 0.23 | 0.24 |
| 2-6 | | 0.16 | 0.19 | 0.23 | 0.24 |
| 2-7 | | 0.15 | 0.20 | 0.23 | 0.25 |
| 2-8 | 40 | 0.17 | 0.19 | 0.24 | 0.25 |
| 2-9 | | 0.15 | 0.19 | 0.23 | 0.24 |
| 2-11 | 60 | 0.17 | 0.17 | 0.23 | 0.26 |
| 2-12 | | 0.16 | 0.17 | 0.23 | 0.26 |
| 2-13 | | 0.15 | 0.18 | 0.22 | 0.25 |
| 2-14 | | 0.16 | 0.20 | 0.24 | 0.26 |

It can be seen from Table 5 that: formulations containing antioxidants and those containing preservatives exhibited essentially identical trends in HMWP changes during long-term storage at 4°C.

**Table 6: HMWP detection results for compound 1 formulations under accelerated conditions at 25°C**

| Formulation No. | Compound 1 (mg/mL) | Day 0 | Day 7 | Day 14 | Day 21 | Day 28 | Day 35 | Day 43 | Day 49 | Day 56 | Day 63 |
|---|---|---|---|---|---|---|---|---|---|---|---|
| 2-1 | 10 | 0.17 | 0.22 | 0.25 | 0.30 | 0.40 | 0.44 | 0.42 | 0.55 | 0.60 | 0.62 |
| 2-2 | | 0.17 | 0.26 | 0.24 | 0.29 | 0.36 | 0.40 | 0.52 | 0.59 | 0.66 | 0.73 |
| 2-3 | | 0.16 | 0.28 | 0.23 | 0.25 | 0.30 | 0.31 | 0.33 | 0.35 | 0.35 | 0.38 |
| 2-4 | | 0.17 | 0.26 | 0.24 | 0.26 | 0.30 | 0.32 | 0.34 | 0.36 | 0.37 | 0.40 |
| 2-5 | 20 | 0.15 | 0.27 | 0.25 | 0.33 | 0.45 | 0.60 | 0.76 | 0.87 | 0.99 | 1.25 |
| 2-6 | | 0.16 | 0.25 | 0.22 | 0.24 | 0.28 | 0.31 | 0.33 | 0.33 | 0.33 | 0.38 |
| 2-7 | | 0.15 | 0.25 | 0.23 | 0.23 | 0.30 | 0.33 | 0.36 | 0.37 | 0.39 | 0.44 |
| 2-8 | 40 | 0.17 | 0.24 | 0.22 | 0.30 | 0.48 | 0.60 | 0.77 | 0.86 | 0.97 | 1.11 |
| 2-9 | | 0.15 | 0.22 | 0.22 | 0.22 | 0.29 | 0.31 | 0.32 | 0.33 | 0.33 | 0.36 |
| 2-10 | | 0.17 | 0.24 | 0.24 | 0.25 | 0.30 | 0.35 | 0.40 | 0.45 | 0.48 | 0.55 |
| 2-11 | 60 | 0.17 | 0.20 | 0.24 | 0.27 | 0.35 | 0.37 | 0.41 | 0.48 | 0.46 | 0.52 |
| 2-12 | | 0.16 | 0.23 | 0.26 | 0.32 | 0.48 | 0.58 | 0.71 | 0.75 | 0.88 | 1.01 |
| 2-13 | | 0.15 | 0.24 | 0.22 | 0.22 | 0.30 | 0.32 | 0.33 | 0.33 | 0.37 | 0.42 |
| 2-14 | | 0.16 | 0.22 | 0.23 | 0.25 | 0.34 | 0.37 | 0.42 | 0.46 | 0.49 | 0.57 |

It can be seen from Table 6 that: all groups exhibited a pronounced increase in HMWP during accelerated strorage at 25°C. In formulations containing sodium chloride, the increase in HMWP decreased with increasing concentrations of compound 1, except in the 10 mg/mL group. This difference was particularly evident at Day 63: I n the compound 1 concentration groups of 10 mg/mL and 60 mg/mL, the increase in HMWP relative to Day 0 was lower when citric acid was added. In the 20 mg/mL and 40 mg/mL groups, the increase in HMWP relative to Day 0 was notably lower in formulations containing citric acid. The addition of antioxidants can slow the increase in HMWP content, and citric acid monohydrate shows a more significant effect than methionine.

**Table 7: Results for HMWP in compound 1 formulations under accelerated conditions at 37°C**

| Formulation No. | Compound 1 (mg/mL) | Day 0 | Day 7 | Day 14 | Day 21 | Day 28 | Day 35 | Day 43 | Day 49 | Day5 6 | Day 63 |
|---|---|---|---|---|---|---|---|---|---|---|---|
| 2-1 | 10 | 0.17 | 0.28 | 0.57 | 0.76 | 1.02 | 1.23 | 1.46 | 1.64 | 1.86 | 2.16 |
| 2-2 | | 0.17 | 0.33 | 0.89 | 1.35 | 1.89 | 2.40 | 3.03 | 3.43 | 4.00 | 4.48 |
| 2-3 | | 0.16 | 0.31 | 0.31 | 0.38 | 0.48 | 0.54 | 0.57 | 0.62 | 0.67 | 0.75 |
| 2-4 | | 0.17 | 0.21 | 0.35 | 0.50 | 0.74 | 0.86 | 1.29 | 1.46 | 1.83 | 2.19 |
| 2-5 | 20 | 0.15 | 0.35 | 0.97 | 1.52 | 2.18 | 2.65 | 3.10 | 3.42 | 3.81 | 3.98 |
| 2-6 | | 0.16 | 0.19 | 0.28 | 0.35 | 0.43 | 0.47 | 0.53 | 0.57 | 0.61 | 0.68 |
| 2-7 | | 0.15 | 0.22 | 0.43 | 0.74 | 1.25 | 1.56 | 1.94 | 2.15 | 2.42 | 2.67 |
| 2-8 | 40 | 0.17 | 0.37 | 1.03 | 1.38 | 1.67 | 1.81 | 2.03 | 2.22 | 2.43 | 2.48 |
| 2-9 | | 0.15 | 0.20 | 0.30 | 0.36 | 0.43 | 0.48 | 0.52 | 0.57 | 0.61 | 0.67 |
| 2-10 | | 0.17 | 0.23 | 0.54 | 0.95 | 1.26 | 1.43 | 1.67 | 1.76 | 1.91 | 2.16 |
| 2-11 | 60 | 0.17 | 0.23 | 0.41 | 0.59 | 0.78 | 0.89 | 1.04 | 1.18 | 1.24 | 1.53 |
| 2-12 | | 0.16 | 0.37 | 0.86 | 1.09 | 1.33 | 1.43 | 1.64 | 1.70 | 1.96 | 2.06 |
| 2-13 | | 0.15 | 0.20 | 0.30 | 0.38 | 0.49 | 0.54 | 0.59 | 0.64 | 0.68 | 0.75 |
| 2-14 | | 0.16 | 0.28 | 0.55 | 0.84 | 1.10 | 1.19 | 1.35 | 1.39 | 1.43 | 1.73 |

It can be seen from Table 7 that: in the accelerated conditions at 37°C, all groups exhibited a pronounced trend of increasing HMWP. As the formulation concentration increased, the levels of HMWP decreased, with a particularly noticeable difference observed at Day 63: In the compound 1 concentration groups of 10 mg/mL and 60 mg/mL, the increase in HMWP relative to Day 0 was lower after citric acid was added. In the 20 mg/mL and 40 mg/mL groups, the increase in HMWP relative to Day 0 was notably lower in the formulations containing citric acid. The addition of antioxidants can slow the growth of HMWP, with citric acid monohydrate proving more effective than methionine.

In summary, the antioxidant citric acid monohydrate can slow the increase in HMWP content in formulation compositions. Among these, citric acid monohydrate demonstrated a more pronounced slowing effect than methionine, thereby being more favorable for improving the stability of the formulations.

Removal of the preservative (phenol) and replacement of the osmotic pressure regulator (propylene glycol → NaCl) result in a significantly increased the levels of related substances and HMWP in the formulations. The addition of antioxidants can markedly reduce the increase in related substances and HMWP, with citric acid monohydrate proving more effective than methionine. Accordingly, citric acid monohydrate was ultimately selected as the antioxidant in the formulations of compound 1.

### Example 3: Determination of Chemical Stability of Formulations

### 3.1 Preparation of Formulations

The stability of formulations was tested using sodium chloride and propylene glycol as osmotic pressure regulators. The formulations was prepared and filled into assembled cartridges. The formulation designs of the osmotic pressure regulator-containing formulations were shown in Table 8:

**Table 8: Compound 1 formulations**

| Formulation No. | Compound 1 (mg/mL) | Na₂HPO₄ (mg/mL) | NaCl (mg/mL) | Phenol (mg/mL) | Propylene glycol (mg/mL) | Citric acid monohydrate (mM) | pH | Nitrogen replacement |
|---|---|---|---|---|---|---|---|---|
| 3-1 | | | - | 5.5 | 14 | - | | - |
| 3-2 | | | | | | | | Replaced |
| 3-3 | | | | - | | | | - |
| 3-4 | | | | | | | | Replaced |
| 3-5 | 30 | 1.42 | | | | 5 | 7.3 | - |
| 3-6 | | | | | | | | Replaced |
| 3-7 | | | 8.25 | | - | - | | - |
| 3-8 | | | | | | | | Replaced |
| 3-9 | | | | | | 5 | | - |
| 3-10 | | | | | | | | Replaced |

### 3.2 Detection of Related Substances

The formulations 3-1 to 3-10 described above were filled into prefilled cartridges and stability studies were conducted at 4°C, 25°C, and 37°C. The increase in related substances at different Days compared to Day 0 were measured for each formulation (formulations 3-1 to 3-10) in Example 3. The test results were shown in Tables 9, 10, and 11, respectively.

**Table 9: Related substances results for compound 1 formulations at 4°C**

| Formulation No. | Day 0 | Day 17 | Day 28 | Day 56 |
|---|---|---|---|---|
| 3-1 | 2.71 | 2.64 | 2.58 | 3.06 |
| 3-2 | 2.76 | 2.67 | 2.70 | 3.08 |
| 3-3 | 2.74 | 2.70 | 2.69 | 3.12 |
| 3-4 | 2.73 | 2.83 | 2.65 | 3.08 |
| 3-5 | 2.78 | 2.74 | 2.72 | 3.15 |
| 3-6 | 2.99 | 2.88 | 2.73 | 3.04 |
| 3-7 | 3.03 | 2.85 | 2.76 | 3.09 |
| 3-8 | 2.86 | 2.91 | 2.81 | 3.09 |
| 3-9 | 2.85 | 2.99 | 2.85 | 3.10 |
| 3-10 | 3.02 | 2.88 | 2.86 | 3.07 |

It can be seen from Table 9 that: the trends in related substances changes were essentially consistent across all groups during long-term storage at 4°C. The two different osmotic pressure regulators had essentially no effect on the stability (related substances content) of compound 1 formulations during long-term 56 days of storage at 4°C.

**Table 10: Content of related substances in compound 1 formulations under accelerated conditions at 25°C**

| Formulation No. | Day 0 | Day 7 | Day 17 | Day 21 | Day 28 | Day 35 | Day 42 | Day 49 | Day 56 |
|---|---|---|---|---|---|---|---|---|---|
| 3-1 | 2.71 | 3.18 | 3.07 | 2.91 | 3.32 | 3.51 | 3.51 | 3.11 | 4.08 |
| 3-2 | 2.76 | 3.27 | 3.18 | 2.93 | 3.26 | 3.55 | 3.45 | 3.06 | 4.14 |
| 3-3 | 2.74 | 3.54 | 3.47 | 3.16 | 3.27 | 4.09 | 4.16 | 4.07 | 5.58 |
| 3-4 | 2.73 | 3.21 | 3.06 | 3.09 | 3.13 | 3.83 | 4.01 | 3.62 | 4.85 |
| 3-5 | 2.78 | 3.14 | 3.00 | 2.93 | 2.85 | 3.50 | 3.39 | 3.08 | 3.91 |
| 3-6 | 2.99 | 3.66 | 2.97 | 2.98 | 2.74 | 3.51 | 3.42 | 2.83 | 3.80 |
| 3-7 | 3.03 | 3.27 | 3.12 | 3.16 | 2.89 | 3.88 | 3.82 | 3.41 | 4.78 |
| 3-8 | 2.86 | 3.26 | 3.11 | 3.09 | 2.95 | 3.80 | 3.69 | 3.23 | 4.45 |
| 3-9 | 2.85 | 3.40 | 3.10 | 3.08 | 2.87 | 3.70 | 3.53 | 3.07 | 3.99 |
| 3-10 | 3.02 | 3.40 | 3.09 | 3.03 | 2.85 | 3.67 | 3.57 | 3.05 | 4.00 |

It can be seen from Table 10 that: the trends in related substances changes during accelerated storage at 25°C were essentially consistent across all groups. It can be observed that sodium chloride, when used as an osmotic pressure regulator, resulted in slower growth of related substances compared to propylene glycol. Furthermore, nitrogen displacement effectively reduced the rate of increase in related substances. Comparisons between groups 3-5 and 3-3, as well as groups 3-9 and 3-7, revealed that the growth rates of related substances in both groups were significantly reduced after citric acid was added. However, nitrogen displacement on the basis of the presence of antioxidants did not significantly affect the reduction in growth rates of these substances.

**Table 11: Results for related substances in compound 1 formulations under accelerated conditions at 37°C**

| Formulation No. | Day 0 | Day 7 | Day 17 | Day 21 | Day 28 | Day 35 | Day 42 | Day 49 | Day 56 |
|---|---|---|---|---|---|---|---|---|---|
| 3-1 | 2.71 | 3.35 | 4.36 | 4.44 | 4.82 | 6.07 | 6.70 | 6.99 | 8.77 |
| 3-2 | 2.76 | 3.37 | 4.28 | 4.45 | 4.77 | 6.10 | 6.55 | 6.64 | 8.44 |
| 3-3 | 2.74 | 3.82 | 5.86 | 6.02 | 6.11 | 7.33 | 7.78 | 8.50 | 10.40 |
| 3-4 | 2.73 | 3.69 | 4.62 | 4.92 | 4.52 | 6.46 | 7.08 | 6.96 | 8.80 |
| 3-5 | 2.78 | 3.35 | 4.34 | 4.54 | 4.76 | 5.98 | 6.13 | 6.13 | 7.83 |
| 3-6 | 2.99 | 3.41 | 3.92 | 4.59 | 4.84 | 5.96 | 5.98 | 6.14 | 7.73 |
| 3-7 | 3.03 | 3.82 | 4.89 | 6.07 | 6.54 | 7.95 | 8.55 | 8.78 | 11.09 |
| 3-8 | 2.86 | 3.70 | 4.42 | 5.00 | 5.52 | 6.87 | 7.11 | 7.56 | 9.40 |
| 3-9 | 2.85 | 3.60 | 4.04 | 4.74 | 5.02 | 6.19 | 6.45 | 6.53 | 8.17 |
| 3-10 | 3.02 | 3.50 | 4.14 | 4.65 | 5.07 | 6.02 | 6.38 | 6.64 | 8.22 |

It can be seen from Table 11 that: the trends in the changes of related substances during accelerated storage at 37°C were essentially consistent across all groups. Comparisons among groups 3-1, 3-3, and 3-7, as well as among groups 3-2, 3-4, and 3-8 revealed that the related substances were increased after phenol was removed. The rate of increase is comparable between formulations using sodium chloride and those using propylene glycol as the osmotic pressure regulator. Nitrogen displacement effectively reduced the rate of increase in related substances. Comparisons between groups 3-5 and 3-3, as well as between groups 3-9 and 3-7, revealed that the growth rates of related substances in groups with different osmotic pressure regulators were both reduced after citric acid was added. Comparison between groups 3-5 and 3-6, and between groups 3-9 and 3-10 revealed that: nitrogen displacement on the basis of the presence of antioxidants did not significantly reduce the rate of increase of related substances.

### 3.3 HMWP Detection

Same to section 2.3, the increase in HMWP relative to Day 0 was measured at 4°C, 25°C, and 37°C for each formulation in Example 3. The results were shown in Tables 12, 13, and 14, respectively.

**Table 12: HMWP results for compound 1 formulations under long-term storage at 4°C**

| Formulation No. | Day 0 | Day 17 | Day 28 | Day 56 |
|---|---|---|---|---|
| 3-1 | 0.25 | 0.26 | 0.26 | 0.27 |
| 3-2 | 0.25 | 0.26 | 0.25 | 0.27 |
| 3-3 | 0.25 | 0.26 | 0.26 | 0.27 |
| 3-4 | 0.26 | 0.27 | 0.27 | 0.27 |
| 3-5 | 0.25 | 0.26 | 0.26 | 0.26 |
| 3-6 | 0.26 | 0.26 | 0.26 | 0.26 |
| 3-7 | 0.25 | 0.25 | 0.27 | 0.28 |
| 3-8 | 0.27 | 0.29 | 0.28 | 0.27 |
| 3-9 | 0.26 | 0.28 | 0.27 | 0.26 |
| 3-10 | 0.26 | 0.29 | 0.27 | 0.30 |

It can be seen from Table 12 that: the trends in HMWP changes during long-term storage at 4°C were essentially consistent across all groups. The two different osmotic pressure regulators had essentially no effect on the stability (HMWP content) of compound 1 formulations during long-term 56 days storage at 4°C.

**Table 13: HMWP content in compound 1 formulations under accelerated conditions at 25°C**

| Formulation No. | Day0 | Day7 | Day17 | Day21 | Day28 | Day35 | Day42 | Day49 | Day56 |
|---|---|---|---|---|---|---|---|---|---|
| 3-1 | 0.25 | 0.27 | 0.32 | 0.29 | 0.36 | 0.39 | 0.46 | 0.46 | 0.51 |
| 3-2 | 0.25 | 0.27 | 0.31 | 0.30 | 0.36 | 0.35 | 0.43 | 0.46 | 0.47 |
| 3-3 | 0.25 | 0.28 | 0.49 | 0.61 | 0.87 | 1.05 | 1.40 | 1.61 | 1.88 |
| 3-4 | 0.26 | 0.31 | 0.47 | 0.50 | 0.70 | 0.80 | 1.02 | 1.20 | 1.42 |
| 3-5 | 0.25 | 0.26 | 0.28 | 0.31 | 0.31 | 0.30 | 0.32 | 0.34 | 0.36 |
| 3-6 | 0.26 | 0.26 | 0.28 | 0.28 | 0.33 | 0.30 | 0.32 | 0.33 | 0.36 |
| 3-7 | 0.25 | 0.27 | 0.34 | 0.36 | 0.55 | 0.60 | 0.79 | 0.90 | 1.07 |
| 3-8 | 0.27 | 0.27 | 0.36 | 0.36 | 0.46 | 0.52 | 0.63 | 0.72 | 0.81 |
| 3-9 | 0.26 | 0.26 | 0.32 | 0.30 | 0.30 | 0.32 | 0.35 | 0.33 | 0.36 |
| 3-10 | 0.26 | 0.26 | 0.31 | 0.30 | 0.30 | 0.32 | 0.37 | 0.34 | 0.35 |

It can be seen from Table 13 that: the trends in changes of HMWP during accelerated storage at 25°C were essentially consistent across all groups. Comparisons among groups 1, 3, and 7, as well as among groups 2, 4, and 8 revealed that removal of phenol resulted in increased HMWP. Among these, the sodium chloride osmotic regulator group exhibited slower growth of HMWP compared to the propylene glycol osmotic regulator group. Nitrogen displacement reduced the increase rate of HMWP. Comparisons between groups 5 and 9 revealed that the increase rate of HMWP in both groups with different osmotic pressure regulators were slowed after citric acid was added. However, nitrogen displacement on the basis of the presence of antioxidants did not significantly reduce the increase of HMWP.

**Table 14: Results for HMWP in compound 1 formulations under accelerated conditions at 37°C**

| Formulation No. | Day 0 | Day 7 | Day 17 | Day 21 | Day 28 | Day 35 | Day 42 | Day 49 | Day 56 |
|---|---|---|---|---|---|---|---|---|---|
| 3-1 | 0.25 | 0.36 | 0.61 | 0.68 | 0.84 | 0.99 | 1.26 | 1.36 | 1.60 |
| 3-2 | 0.25 | 0.34 | 0.53 | 0.60 | 0.70 | 0.81 | 0.99 | 1.13 | 1.28 |
| 3-3 | 0.25 | 0.87 | 1.91 | 2.01 | 2.25 | 2.41 | 2.69 | 2.86 | 3.13 |
| 3-4 | 0.26 | 0.70 | 1.03 | 1.19 | 1.37 | 1.47 | 1.76 | 1.86 | 2.18 |
| 3-5 | 0.25 | 0.32 | 0.38 | 0.41 | 0.45 | 0.46 | 0.59 | 0.60 | 0.65 |
| 3-6 | 0.26 | 0.30 | 0.37 | 0.39 | 0.43 | 0.46 | 0.51 | 0.54 | 0.55 |
| 3-7 | 0.25 | 0.55 | 1.42 | 1.71 | 2.16 | 2.45 | 2.82 | 2.99 | 3.30 |
| 3-8 | 0.27 | 0.47 | 1.14 | 1.11 | 1.36 | 1.46 | 1.78 | 1.92 | 2.21 |
| 3-9 | 0.26 | 0.34 | 0.43 | 0.38 | 0.45 | 0.49 | 0.59 | 0.61 | 0.63 |
| 3-10 | 0.26 | 0.36 | 0.41 | 0.39 | 0.43 | 0.45 | 0.52 | 0.51 | 0.56 |

Among them, formulations containing sodium chloride as the osmotic pressure regulator exhibit a slightly slower increase than those containing propylene glycol as the osmotic pressure regulator within the first 28 days. A comparison between Groups 3-5 and 3-9 indicates that, in formulations containing different osmotic pressure regulators, the addition of citric acid reduces the rate of increase of HMWP in both groups.

In summary, formulations containing sodium chloride as an osmotic pressure regulator demonstrated superior stability at accelerated conditions (25°C) compared to those using propylene glycol as the osmotic pressure regulator. The addition of the antioxidant citric acid monohydrate significantly reduced the increase in related substances and HMWP, exhibiting better stability in accelerated stability testing than formulations containing the preservative phenol.

### Example 4: Effect of Devices on Formulation Stability

### 4.1 Preparation of Formulations

The compound 1 was dissolved in a disodium hydrogen phosphate solution to the final concentrations specified in Table 15. According to the amounts of each component listed in the table below, each excipient solution was added sequentially, and the pH was adjusted to the values indicated in the table below. The final formulations as shown in Table 15 were produced and filled into corresponding devices, namely assembled cartridge, pre-filled cartridge, and pre-filled syringe.

**Table 15: Formulations for the compound 1**

| Formulation No. | Compound 1 (mg/mL) | Na₂HPO₄ (mg/mL) | NaCl (mg/mL) | Phenol (mg/mL) | Propylene glycol (mg/mL) | Citric acid monohydrate (mM) | pH | Devices |
|---|---|---|---|---|---|---|---|---|
| 4-1 | 2 | 1.42 | - | 5.5 | 14 | - | 7.3 | Assemble cartridge |
| 4-2 | 48 | | | | | | | |
| 4-3 | 2 | | 5.5 | - | - | 5 | | Pre-filled cartridge |
| 4-4 | | | | | | | | Pre-filled syringe |
| 4-5 | 48 | | | | | | | Pre-filled cartridge |
| 4-6 | | | | | | | | Pre-filled syringe |

### 4.2 Detection of Related Substances

The formulations 4-1 to 4-6 described above were filled into their corresponding devices, and subjected to stability studies at 4°C, 25°C, and 37°C. The increase in related substances relative to Day 0 for each formulation listed in Table 15 was measured at different time points. The test results are shown in Tables 16, 17, and 18, respectively.

**Table 16: Related substances results for compound 1 formulations under long-term storage conditions at 4°C**

| Formulation No. | Compound 1 (mg/mL) | Day 0 | Day 14 | Day 28 |
|---|---|---|---|---|
| 4-1 | 2 | 1.85 | 1.98 | 1.99 |
| 4-2 | 48 | 1.80 | 1.93 | 1.91 |
| 4-3 | 2 | 1.82 | 1.96 | 2.05 |
| 4-4 | 2 | 1.82 | 2.02 | 2.02 |
| 4-5 | 48 | 1.83 | 1.92 | 2.04 |
| 4-6 | 48 | 1.83 | 1.92 | 1.97 |

It can be seen from Table 16 that: the change trends in related substances for each group were substantially consistent during long-term storage at 4°C. There were no significant differences in related substances between groups, and different devices had no significant effects on the stability of Compound 1 formulations.

**Table 17: Comparison of related substances results for compound 1 formulations under accelerated conditions at 25°C**

| Formulation No. | Compound 1 (mg/mL) | Day 0 | Day 7 | Day 14 | Day 21 | Day 28 |
|---|---|---|---|---|---|---|
| 4-2 | 48 | 1.80 | 2.06 | 2.24 | 2.55 | 2.40 |
| 4-5 | 48 | 1.83 | 1.98 | 2.14 | 2.34 | 2.44 |
| 4-6 | 48 | 1.83 | 1.96 | 2.11 | 2.45 | 2.45 |

It can be seen from Table 17 that: different devices had no significant effects on the stability of Compound 1 formulations.

**Table 18: Related substances results for compound 1 formulations under accelerated conditions at 37°C**

| Formulation No. | Compound1 (mg/mL) | Day 0 | Day7 | Day14 | Day21 | Day28 |
|---|---|---|---|---|---|---|
| 4-2 | 48 | 1.80 | 2.47 | 3.15 | 3.86 | 4.55 |
| 4-3 | 2 | 1.82 | 2.63 | 3.19 | 3.95 | 4.55 |
| 4-6 | 48 | 1.83 | 2.47 | 3.17 | 3.81 | 4.63 |

It can be seen from Table 18 that: different devices had no significant effects on the stability of Compound 1 formulations.

### 4.3 Detection of HMWP

Similar to Section 4.2, the increase in HMWP relative to Day 0 was measured at 4°C, 25°C, and 37°C for each formulation in Table 18. The test results are shown in Tables 19, 20, and 21, respectively.

**Table 19: HMWP content of compound 1 formulations under long-term conditions at 4°C in comparative experiment**

| Formulation No. | Compound 1 (mg/mL) | Day 0 | Day 14 | Day 28 |
|---|---|---|---|---|
| 4-1 | 2 | 0.26 | 0.25 | 0.28 |
| 4-2 | 48 | 0.25 | 0.23 | 0.27 |
| 4-3 | 2 | 0.24 | 0.23 | 0.27 |
| 4-4 | 2 | 0.24 | 0.23 | 0.23 |
| 4-5 | 48 | 0.26 | 0.27 | 0.22 |
| 4-6 | 48 | 0.26 | 0.23 | 0.26 |

It can be seen from Table 19 that: different devices had no significant effect on the stability (HMWP content) of Compound 1 formulations during long-term storage at 4°C for 28 days.

**Table 20: Changes of HMWP in compound 1 formulations under accelerated conditions at 25°C**

| Formulation No. | Compound 1 (mg/mL) | Day 0 | Day 7 | Day 14 | Day 21 | Day 28 |
|---|---|---|---|---|---|---|
| 4-3 | 2 | 0.24 | 0.28 | 0.27 | 0.29 | 0.35 |
| 4-4 | 2 | 0.24 | 0.30 | 0.30 | 0.38 | 0.29 |
| 4-5 | 48 | 0.26 | 0.29 | 0.29 | 0.34 | 0.28 |
| 4-6 | 48 | 0.26 | 0.29 | 0.26 | 0.29 | 0.33 |

It can be seen from Table 20 that: different devices had no significant effect on the stability of Compound 1 formulations.

**Table 21: HMWP results under accelerated conditions at 37°C for compound 1 injection solution devices in comparison experiment**

| Formulation No. | Compound 1 (mg/mL) | Day 0 | Day 7 | Day 14 | Day 21 | Day 28 |
|---|---|---|---|---|---|---|
| 4-4 | 2 | 0.24 | 0.41 | 0.35 | 0.57 | 0.49 |
| 4-5 | 48 | 0.26 | 0.33 | 0.37 | 0.47 | 0.39 |
| 4-6 | 48 | 0.26 | 0.33 | 0.35 | 0.43 | 0.48 |

It can be seen from Table 21 that: comparing Groups 4-3 with 4-4, as well as Groups 4-5 with 4-6, the change trends in HMWP for each group were essentially consistent during accelerated storage at 37°C: There were no significant differences in HMWP between pre-filled cartridges and pre-filled syringes. The changes in related substances of Groups 4-3 and 4-4 were slightly superior to those of Group 4-1. Comparing Groups 4-5 and 4-6 with group 4-2, no significant differences were observed.

### Example 5: Effect of GLP-1 Peptide Concentration on Formulation Stability

### 5.1 Preparation of Formulations

The concentrations of Compound 1 in the formulations were divided into four concentration levels (6 mg/mL, 12 mg/mL, 24 mg/mL, and 48 mg/mL), with a sodium chloride concentration of 6 mg/mL. Stability studies were conducted on formulations of Compound 1 at these four concentration levels. Nitrogen replacement was not performed for any of the experiment groups. The experimental design for the formulation concentration confirmation is shown in Table 22 below:

**Table 22: Compound 1 formulations**

| Formulations | Compound 1 (mg/mL) | Na₂HPO₄ (mg/mL) | NaCl (mg/mL) | Phenol (mg/mL) | Propylene glycol (mg/mL) | Citric acid monohydrate | pH |
|---|---|---|---|---|---|---|---|
| 5-1 | 5 | 1.42 | - | 5.5 | 14 | - | 7.3 |
| 5-2 | 30 | | | - | | | |
| 5-3 | 6 | | 6 | | - | 5mM | |
| 5-4 | 12 | | | | | | |
| 5-5 | 24 | | | | | | |
| 5-6 | 48 | | | | | | |

### 5.2 Detection of Related Substances

The above-mentioned formulations 5-1 to 5-6 were filled into their corresponding devices and subjected to stability studies at 4°C, 25°C, and 37°C. The increase in related substances over different days relative to Day 0 was measured for each formulation listed in Table 22, as shown in Tables 23, 24, and 25 respectively.

**Table 23: Related substances results for compound 1 formulations under long-term storage conditions at 4°C**

| Formulations | Compound 1 (mg/mL) | Day 0 | Day 14 | Day 28 |
|---|---|---|---|---|
| 5-1 | 5 | 1.95 | 2.05 | 1.90 |
| 5-2 | 30 | 1.95 | 2.15 | 1.80 |
| 5-3 | 6 | 2.01 | 2.13 | 1.87 |
| 5-4 | 12 | 1.94 | 2.07 | 1.78 |
| 5-5 | 24 | 2.06 | 2.27 | 2.03 |
| 5-6 | 48 | 2.16 | 2.11 | 2.16 |

It can be seen from Table 23 that: the change trends in related substances for each group were essentially consistent during long-term storage at 4°C. Different formulations had no significant effect on the stability (related substances content) of Compound 1 formulations during long-term storage at 4°C for 28 days.

**Table 24: Related substances results for compound 1 formulations under accelerated conditions at 25°C**

| Formulations | Compound 1 (mg/mL) | Day 0 | Day 7 | Day 14 | Day 21 | Day 28 |
|---|---|---|---|---|---|---|
| 5-3 | 6 | 2.01 | 2.02 | 2.24 | 2.09 | 2.51 |
| 5-4 | 12 | 1.94 | 2.07 | 2.23 | 2.14 | 2.49 |
| 5-5 | 24 | 2.06 | 2.05 | 2.23 | 2.12 | 2.45 |
| 5-6 | 48 | 2.16 | 2.15 | 2.27 | 2.21 | 2.52 |

It can be seen from Table 24 that: the change trends in related substances among the groups were essentially consistent during accelerated storage at 25°C. No significant differences in related substances were observed among Groups 5-3 to 5-6.

**Table 25: Related substances results for compound 1 formulations under accelerated conditions at 37°C**

| Formulations | Compound 1 (mg/mL) | Day 0 | Day 7 | Day 14 | Day 21 | Day 28 |
|---|---|---|---|---|---|---|
| 5-1 | 5 | 1.95 | 2.42 | 3.28 | 3.83 | 4.67 |
| 5-3 | 6 | 2.01 | 2.48 | 3.20 | 3.69 | 4.87 |
| 5-4 | 12 | 1.94 | 2.44 | 3.24 | 3.59 | 4.65 |
| 5-5 | 24 | 2.06 | 2.61 | 3.25 | 3.60 | 4.58 |
| 5-6 | 48 | 2.16 | 2.52 | 3.30 | 3.82 | 4.73 |

It can be seen from Table 25 that: related substances in each group exhibited an increasing trend during accelerated storage at 37°C. No significant differences in related substances were observed among the groups.

### 5.3 Detection of HMWP

Similar to Section 5.2, the increase in HMWP relative to Day 0 was measured at 4°C, 25°C, and 37°C for each formulation in Table 22. The detection results are shown in Tables 26, 27, and 28, respectively.

**Table 26: HMWP results for compound 1 formulations at 4°C**

| Formulations | Compound 1 (mg/mL) | Day 0 | Day 14 | Day 28 |
|---|---|---|---|---|
| 5-1 | 5 | 0.22 | 0.26 | 0.26 |
| 5-2 | 30 | 0.22 | 0.26 | 0.26 |
| 5-3 | 6 | 0.22 | 0.27 | 0.26 |
| 5-4 | 12 | 0.23 | 0.26 | 0.27 |
| 5-5 | 24 | 0.22 | 0.25 | 0.25 |
| 5-6 | 48 | 0.23 | 0.26 | 0.25 |

It can be seen from Table 26 that: the change trends in HMWP for each group were essentially consistent during long-term storage at 4°C. Different formulations had no significant effect on the stability (HMWP content) of Compound 1 formulations during 28 days of long-term storage at 4°C.

**Table 27: HMWP results for compound 1 formulation concentration confirmation experiments under 25°C accelerated conditions**

| Formulations | Compound 1 (mg/mL) | Day 0 | Day 7 | Day 14 | Day 21 | Day 28 |
|---|---|---|---|---|---|---|
| 5-1 | 5 | 0.22 | 0.29 | 0.33 | 0.42 | 0.37 |
| 5-3 | 6 | 0.22 | 0.28 | 0.33 | 0.34 | 0.32 |
| 5-4 | 12 | 0.23 | 0.28 | 0.31 | 0.32 | 0.31 |
| 5-5 | 24 | 0.22 | 0.27 | 0.30 | 0.32 | 0.31 |
| 5-6 | 48 | 0.23 | 0.26 | 0.30 | 0.32 | 0.30 |

It can be seen from Table 27 that: HMWP in each group exhibited an increasing trend during accelerated storage at 25°C, with no significant differences observed among them.

**Table 28: HMWP results for compound 1 formulation concentration confirmation experiment under 37°C accelerated conditions**

| Formulations | Compound 1 (mg/mL) | Day 0 | Day 7 | Day 14 | Day 21 | Day 28 |
|---|---|---|---|---|---|---|
| 5-3 | 6 | 0.22 | 0.38 | 0.46 | 0.57 | 0.55 |
| 5-4 | 12 | 0.23 | 0.32 | 0.42 | 0.48 | 0.51 |
| 5-5 | 24 | 0.22 | 0.33 | 0.42 | 0.49 | 0.47 |
| 5-6 | 48 | 0.23 | 0.32 | 0.40 | 0.46 | 0.49 |

It can be seen from Table 28 that: related substances in each group exhibited an increasing trend during accelerated storage at 37°C, with no significant differences observed among them.

### Example 6: Effect of Osmotic Pressure Regulator Concentration on Formulation

In the compound 1 formulations, the osmotic pressure regulator sodium chloride was primarily used to regulate the osmotic pressure within the formulations. Varying concentrations of the compound 1 (with all other excipients at identical concentrations) resulted in differing osmotic pressures. The osmotic pressure at the maximum formulation concentration of 48 mg/mL approximates the upper limit of normal human osmotic pressure (280-310 mOsmol/kg), specifically 310 mOsmol/kg. The osmotic pressure at the minimum formulation concentration of 6 mg/mL approximates the lower limit of normal human osmotic pressure (280 mOsmol/kg). No nitrogen displacement or packaging was performed in the experiments. The osmotic pressure regulator concentration design and results are shown in Table 29:

**Table 29: Compound 1 formulations and osmotic pressure results**

| Formulation No. | NaCl (mg/mL) | Compound1 (mg/mL) | Na₂HPO₄ (mg/mL) | Citric acid monohydrate (mg/mL) | pH | Osmotic pressure (mOsmol/kg) |
|---|---|---|---|---|---|---|
| 6-1 | 6 | 0 | 1.42 | 1.05 (5mM) | 7.3 | 235 |
| 6-2 | | 6 | | | | 239 |
| 6-3 | | 12 | | | | 253 |
| 6-4 | | 24 | | | | 260 |
| 6-5 | | 48 | | | | 285 |
| 6-6 | 7 | 0 | 1.42 | 1.05 (5mM) | 7.3 | 268 |
| 6-7 | | 6 | | | | 273 |
| 6-8 | | 12 | | | | 283 |
| 6-9 | | 24 | | | | 294 |
| 6-10 | | 48 | | | | 320 |

As shown in the table above, the osmotic pressure results indicated that at a sodium chloride concentration of 7 mg/mL, the osmotic pressure of the minimum formulation concentration of 6 mg/mL is 273 mOsmol/kg, approaching the minimum normal human osmotic pressure of 280 mOsmol/kg. The osmotic pressure of the formulations at the maximum concentration of 48 mg/mL is 320 mOsmol/kg, approaching the upper limit of normal human osmolarity of 310 mOsmol/kg.

### Example 7: Effect of Citric Acid Concentration on Formulation Stability

Citric acid in formulations can cause injection site pain. Reducing citric acid concentration can alleviate pain induced by citric acid (Ya Lan Yang1 and Ted Weita Lai1,2,3. Citric Acid in Drug Formulations Causes Pain by Potentiating Acid-Sensing Ion Channel 1 [J]. The Journal of Neuroscience, May 26, 2021•41(21):4596-4606). Therefore, it is necessary to identify an optimal citric acid concentration that minimizes pain while maintaining good stability.

### 7.1 Study on the Effect of Citric Acid Concentration (0.005 mM-10 mM) on Formulation Stability

The effect of citric acid concentrations ranging from 0.005 mM to 10 mM on formulation stability was investigated. A formulation of 2 mg/mL Compound 1 (with other components identical to Formulation 5-1) was selected for stability testing under accelerated conditions at 37°C.

**Table 30: Effect of 0.005 mM-0.1 mM citric acid on stability (HMWP content)**

| Formulation No. | Formulations | Day 0 | Day 7 | Day 14 | Day 21 | Day 28 | Day 36 | Day 42 | Day 49 | Day 56 | Day 65 | Day 70 |
|---|---|---|---|---|---|---|---|---|---|---|---|---|
| 7-1 | 0 mM Citric acid | 0.3 | 0.61 | 0.97 | 1.25 | 1.58 | 2.09 | 2.12 | 2.76 | 2.66 | 3.12 | 3.36 |
| 7-2 | 0.1 mM Citric acid | 0.32 | 0.53 | 1.07 | 0.93 | 1.14 | 1.47 | 1.54 | 1.76 | 1.87 | 2.11 | 2.25 |
| 7-3 | 0.05 mM Citric acid | 0.29 | 0.53 | 0.84 | 1.02 | 1.21 | 1.62 | 1.84 | 1.96 | 2.21 | 2.51 | 2.64 |
| 7-3 | 0.01 mM Citric acid | 0.29 | 0.59 | 0.89 | 1.14 | 1.35 | 2.23 | 1.96 | 2.4 | 2.5 | 2.79 | 2.98 |
| 7-4 | 0.005 mM Citric acid | 0.31 | 0.6 | 0.9 | 1.21 | 1.42 | 2.32 | 2.16 | 2.41 | 2.54 | 3.17 | 3.19 |

**Table 31: Effect of 0.1 mM-1 mM citric acid on stability (HMWP content)**

| Formulations | Day 0 | Day 7 | Day 14 |
|---|---|---|---|
| 0 mM Citric acid | 0.31 | 1.29 | 2.09 |
| 0.1mM Citric acid | 0.34 | 1.01 | 1.63 |
| 0.2mM Citric acid | 0.34 | 0.94 | 1.51 |
| 0.5mM Citric acid | 0.33 | 0.74 | 1.31 |
| 1mM Citric acid | 0.34 | 0.73 | 1.24 |

**Table 32: Effect of 1 mM-10 mM citric acid on stability (HMWP content)**

| Formulations | Day 0 | Day 7 | Day 14 | Day 18 | Day 35 | Day 41 | Day 49 | Day 55 | Day 63 | Day 69 |
|---|---|---|---|---|---|---|---|---|---|---|
| 0mM Citric acid | 0.25 | 1.16 | 2.02 | 2.45 | 3.68 | 4.36 | 4.48 | 4.79 | 5.25 | 5.82 |
| 1mM Citric acid | 0.27 | 0.62 | 1.15 | 1.4 | 2.11 | 2.59 | 2.64 | 2.78 | 3.06 | 3.57 |
| 2mM Citric acid | 0.28 | 0.62 | 1.15 | 1.37 | 2.06 | 2.65 | 2.55 | 2.9 | 3.02 | 3.47 |
| 5mM Citric acid | 0.29 | 0.8 | 1.23 | 1.43 | 2.07 | 2.73 | 2.6 | 2.8 | 3.06 | 3.46 |
| 10mM Citric | 0.26 | 0.8 | 1.18 | 1.46 | 2.04 | 2.71 | 2.5 | 2.77 | 2.97 | 3.44 |
| acid | | | | | | | | | | |

**Table 33: Effect of 1 mM-10 mM citric acid on stability (related substances content)**

| Formulations | Day 0 | Day 7 | Day14 | Day 18 | Day 35 | Day 41 | Day 49 | Day 55 | Day 63 | Day 69 |
|---|---|---|---|---|---|---|---|---|---|---|
| 0mM Citric acid | 97.42 | 93.49 | 92.19 | 91.68 | 87.83 | 88.09 | 87.20 | 87.06 | 84.44 | 84.28 |
| 1mM Citric acid | 97.64 | 93.97 | 92.93 | 92.44 | 90.23 | 89.74 | 88.64 | 88.78 | 87.85 | 86.36 |
| 2mM Citric acid | 97.59 | 93.69 | 92.75 | 92.43 | 90.35 | 89.70 | 88.80 | 88.90 | 87.87 | 86.45 |
| 5mM Citric acid | 97.57 | 93.58 | 92.48 | 92.34 | 90.09 | 89.55 | 88.63 | 88.75 | 87.75 | 86.33 |
| 10mM Citric acid | 97.63 | 93.48 | 92.49 | 92.09 | 90.19 | 89.05 | 88.33 | 88.49 | 87.73 | 86.48 |

The three experiments above demonstrate that at citric acid concentrations ranging from 0.005 mM to 1 mM, the growth trend of HMWP decreases as citric acid concentration increases; there is no effect on the changes in related substances. At citric acid concentrations ranging from 1 mM to 10 mM, the addition of citric acid reduces the growth trends of related substances and HMWP, but this reduction does not change with increasing citric acid concentration.

### 7.2 Study on the effect of citric acid on formulation stability

| Prescription excipients | Compound 1 Formulations | | | | |
|---|---|---|---|---|---|
| | 7.2-1 | 7.2-2 | 7.2-3 | 7.2-4 | 7.2-5 |
| Phenol | 5.5mg/mL | - | - | - | - |
| Propylene glycol | 14mg/mL | 14mg/mL | 14mg/mL | - | - |
| Sodium chloride | - | - | - | 7mg/mL | 7mg/mL |
| Citric acid monohydrate | - | - | 5mM | - | 5mM |
| Anhydrous disodium hydrogen phosphate | 1.42mg/mL | | | | |

The stability of the formulations containing Compound 1 at a concentration of 2 mg/mL was evaluated under accelerated conditions at 37°C. Accelerated stability studies (HMWP content) were conducted for each formulation at 37°C, with results shown in Tables 34 and 35.

**Table 34: Effect of 5 mM citric acid on formulation stability (HMWP content)**

| Formulation No. | Day0 | Day7 | Day 17 | Day 21 | Day 28 | Day 35 | Day 42 | Day 49 | Day 56 |
|---|---|---|---|---|---|---|---|---|---|
| 7.2-1 | 0.25 | 0.36 | 0.61 | 0.68 | 0.84 | 0.99 | 1.26 | 1.36 | 1.60 |
| 7.2-2 | 0.25 | 0.87 | 1.91 | 2.01 | 2.25 | 2.41 | 2.69 | 2.86 | 3.13 |
| 7.2-3 | 0.25 | 0.32 | 0.38 | 0.41 | 0.45 | 0.46 | 0.59 | 0.60 | 0.65 |
| 7.2-4 | 0.25 | 0.55 | 1.42 | 1.71 | 2.16 | 2.45 | 2.82 | 2.99 | 3.30 |
| 7.2-5 | 0.26 | 0.34 | 0.43 | 0.38 | 0.45 | 0.49 | 0.59 | 0.61 | 0.63 |

**Table 35: Effect of 5 mM citric acid on the stability (related substances content) of Each Formulation**

| Formulations | Day 0 | Day 7 | Day 17 | Day 21 | Day 28 | Day 35 | Day 42 | Day 49 | Day 56 |
|---|---|---|---|---|---|---|---|---|---|
| 7.2-1 | 0.25 | 0.36 | 0.61 | 0.68 | 0.84 | 0.99 | 1.26 | 1.36 | 1.60 |
| 7.2-2 | 0.25 | 0.87 | 1.91 | 2.01 | 2.25 | 2.41 | 2.69 | 2.86 | 3.13 |
| 7.2-3 | 0.25 | 0.32 | 0.38 | 0.41 | 0.45 | 0.46 | 0.59 | 0.60 | 0.65 |
| 7.2-4 | 0.25 | 0.55 | 1.42 | 1.71 | 2.16 | 2.45 | 2.82 | 2.99 | 3.30 |
| 7.2-5 | 0.26 | 0.34 | 0.43 | 0.38 | 0.45 | 0.49 | 0.59 | 0.61 | 0.63 |

It can be seen from Tables 34 and 35 above that citric acid concentrations ranging from 1 mM to 10 mM contribute to enhancing the stability of Compound 1 formulations, with no significant difference observed in their stabilizing effects across this concentration range.

### 7.3 Effect of Citric Acid Concentration on the Stability of Compound 1 Formulations at Different Concentrations 7.3.1 Preparation of Formulations

Formulations of the compound 1 at low (3 mg/mL) and high (48 mg/mL) concentrations were selected for citric acid concentration studies. Formulations 1-10 were prepared as shown in Table 36 below.

**Table 36: Formulations of the compound 1**

| Formulation No. | Compound 1 (mg/mL) | Citric acid monohydrate (mM) | Na₂HPO₄ (mg/mL) | NaCl (mg/mL) | pH |
|---|---|---|---|---|---|
| 7.3-1 | 3 | 0 | | | |
| 7.3-2 | | 0.5 | | | |
| 7.3-3 | | 1 | | | |
| 7.3-4 | | 2 | | | |
| 7.3-5 | | 5 | 1.42 | 7 | 7.3 |
| 7.3-6 | 48 | 0 | | | |
| 7.3-7 | | 0.5 | | | |
| 7.3-8 | | 1 | | | |
| 7.3-9 | | 2 | | | |
| 7.3-10 | | 5 | | | |

### 7.3.2 Detection of Related Substances

The formulations 7.3-1 to 7.3-10 described above were filled into prefilled cartridges and subjected to stability studies at 4°C, 25°C, and 37°C. The increase in related substances relative to Day 0 was measured for each formulation (formulations 7.3-1 to 7.3-10) at different days. The test results are shown in Tables 37, 38, and 39.

**Table 37: Related substances detection results for compound 1 formulations under long-term storage conditions at 4°C**

| Formulation No. | Compound 1 (mg/mL) | Citric acid monohydrate (mM) | Day0 | Day29 | Day55 |
|---|---|---|---|---|---|
| 7.3-1 | 3 | 0 | 2.27 | 2.23 | 2.33 |
| 7.3-2 | | 0.5 | 2.21 | 2.27 | 2.32 |
| 7.3-3 | | 1 | 2.22 | 2.27 | 2.17 |
| 7.3-4 | | 2 | 2.26 | 2.25 | 2.23 |
| 7.3-5 | | 5 | 2.22 | 2.26 | 2.25 |
| 7.3-6 | 48 | 0 | 2.18 | 2.20 | 2.22 |
| 7.3-7 | | 0.5 | 2.18 | 2.20 | 2.29 |
| 7.3-8 | | 1 | 2.15 | 2.27 | 2.30 |
| 7.3-9 | | 2 | 2.21 | 2.19 | 2.25 |
| 7.3-10 | | 5 | 2.17 | 2.25 | 2.20 |

It can be seen from Table 37 that: the change trends in related substances for each group were essentially consistent during long-term storage at 4°C. Over 55 days of long-term storage at 4°C, different concentrations of citric acid monohydrate had no significant effect on the stability trend (changes in related substances content) of Compound 1 formulation.

**Table 38: Detection results of related substances for compound 1 formulations under accelerated conditions at 25°C**

| Formulation No. | Compound 1(mg/mL) | Citric acid monohydrate (mM) | Day 0 | Day 29 | Day 55 |
|---|---|---|---|---|---|
| 7.3-1 | 3 | 0 | 2.27 | 2.95 | 3.29 |
| 7.3-2 | | 0.5 | 2.21 | 2.83 | 3.11 |
| 7.3-3 | | 1 | 2.22 | 2.58 | 2.88 |
| 7.3-4 | | 2 | 2.26 | 2.85 | 2.99 |
| 7.3-5 | | 5 | 2.22 | 2.76 | 3.05 |
| 7.3-6 | 48 | 0 | 2.18 | 3.25 | 4.19 |
| 7.3-7 | | 0.5 | 2.18 | 3.15 | 3.86 |
| 7.3-8 | | 1 | 2.15 | 2.92 | 3.50 |
| 7.3-9 | | 2 | 2.21 | 2.84 | 3.25 |
| 7.3-10 | | 5 | 2.17 | 3.05 | 3.14 |

It can be seen from table 38 that: in formulations containing 48 mg/mL of Compound 1, the growth rate of related substances decreases as the concentration of citric acid monohydrate increases. In formulations containing 3 mg/mL of Compound 1, citric acid monohydrate concentrations ranging from 0.5 mM to 5 mM contribute to decreasing the growth of related substances compared to formulations without citric acid monohydrate. However, the effect at 0.5 mM citric acid monohydrate is weaker than that at1 mM to 5 mM.

**Table 39: Detection results of related substances for compound 1 formulations under accelerated conditions at 37°C**

| Formulation No. | Compound 1 (mg/mL) | Citric acid monohydrate(mM) | Day0 | Day7 | Day14 | Day22 | Day29 | Day55 |
|---|---|---|---|---|---|---|---|---|
| 7.3-1 | 3 | 0 | 2.27 | 3.10 | 4.42 | 5.34 | 7.27 | 13.54 |
| 7.3-2 | | 0.5 | 2.21 | 2.92 | 3.72 | 3.92 | 5.07 | 7.56 |
| 7.3-3 | | 1 | 2.22 | 2.91 | 3.57 | 3.77 | 5.01 | 7.40 |
| 7.3-4 | | 2 | 2.26 | 2.89 | 3.67 | 4.01 | 4.83 | 7.32 |
| 7.3-5 | | 5 | 2.22 | 2.86 | 3.65 | 3.98 | 5.17 | 7.33 |
| 7.3-6 | 48 | 0 | 2.18 | 3.10 | 4.58 | 4.91 | 6.22 | 8.81 |
| 7.3-7 | | 0.5 | 2.18 | 2.92 | 4.28 | 4.83 | 5.85 | 8.60 |
| 7.3-8 | | 1 | 2.15 | 2.81 | 4.03 | 4.59 | 6.33 | 8.38 |
| 7.3-9 | | 2 | 2.21 | 2.79 | 3.87 | 4.34 | 5.47 | 8.15 |
| 7.3-10 | | 5 | 2.17 | 2.82 | 3.79 | 4.08 | 5.26 | 7.40 |

It can be seen from Table 39 that: in formulations containing 3 mg/mL of Compound 1, the stability of related substances in formulations with citric acid monohydrate significantly superior to those without citric acid. Formulations with 2-5 mM citric acid monohydrate show similar growth trends in related substances, all exhibiting greater stability than thant of the formulations with 0.5 mM citric acid monohydrate; In formulations containing 48 mg/mL of Compound 1, the growth rate of related substances decreases as the concentration of citric acid monohydrate increases.

### 7.3.3 Detection of HMWP

Similar to Section 7.3.2, the increase in HMWP relative to Day 0 was measured at 4°C, 25°C, and 37°C for formulations in Example 7.3.1. The detection results are shown in Tables 40, 41, and 42, respectively.

**Table 40: Detection results of HMWP content for compound 1 formulations under long-term storage conditions at 4°C**

| Formulation No. | Compound 1(mg/mL) | Citric acid monohydrate (mM) | Day 0 | Day 29 | Day 55 |
|---|---|---|---|---|---|
| 7.3-1 | 3 | 0 | 0.29 | 0.32 | 0.29 |
| 7.3-2 | | 0.5 | 0.32 | 0.36 | 0.31 |
| 7.3-3 | | 1 | 0.31 | 0.32 | 0.34 |
| 7.3-4 | | 2 | 0.33 | 0.35 | 0.31 |
| 7.3-5 | | 5 | 0.32 | 0.33 | 0.31 |
| 7.3-6 | 48 | 0 | 0.34 | 0.32 | 0.30 |
| 7.3-7 | | 0.5 | 0.32 | 0.31 | 0.34 |
| 7.3-8 | | 1 | 0.33 | 0.31 | 0.31 |
| 7.3-9 | | 2 | 0.31 | 0.31 | 0.30 |
| 7.3-10 | | 5 | 0.31 | 0.30 | 0.32 |

It can be seen from Table 40 that: the change trends in HMWP for each formulation were essentially consistent during long-term storage at 4°C. Different concentrations of citric acid monohydrate had no significant effect on the growth rate of HMWP in compound 1 formulations during the 55 days of long-term storage at 4°C.

**Table 41: HMWP detection results for compound 1 formulations under accelerated conditions at 25°C**

| Formulation No. | Compound 1(mg/mL) | Citric acid monohydrate (mM) | Day 0 | Day 29 | Day 55 |
|---|---|---|---|---|---|
| 7.3-1 | 3 | 0 | 0.29 | 0.43 | 0.69 |
| 7.3-2 | | 0.5 | 0.32 | 0.42 | 0.44 |
| 7.3-3 | | 1 | 0.31 | 0.43 | 0.45 |
| 7.3-4 | | 2 | 0.33 | 0.40 | 0.46 |
| 7.3-5 | | 5 | 0.32 | 0.41 | 0.45 |
| 7.3-6 | 48 | 0 | 0.34 | 0.83 | 1.41 |
| 7.3-7 | | 0.5 | 0.32 | 0.56 | 1.06 |
| 7.3-8 | | 1 | 0.33 | 0.45 | 0.74 |
| 7.3-9 | | 2 | 0.31 | 0.42 | 0.52 |
| 7.3-10 | | 5 | 0.31 | 0.39 | 0.45 |

It can be seen from Table 41 that: in formulations containing 3 mg/mL of Compound 1 with different concentrations (0.5 mM-5 mM) of citric acid monohydrate, the growth rates of HMWP were essentially consistent, and all were superior to that of the formulation 7.3-1 without citric acid monohydrate. In formulations containing 48 mg/mL of Compound 1, the growth rate of HMWP decreases as the citric acid monohydrate concentration increases.

**Table 42: Detection results of HMWP for compound 1 formulations under accelerated conditions at 37°C**

| Formulation No. | Compound 1 (mg/mL) | Citric acid monohydrate mM | Day 0 | Day 7 | Day 14 | Day 22 | Day 29 | Day 55 |
|---|---|---|---|---|---|---|---|---|
| 7.3-1 | 3 | 0 | 0.29 | 0.6 | 1.18 | 1.82 | 2.57 | 5.15 |
| 7.3-2 | | 0.5 | 0.32 | 0.40 | 0.50 | 0.58 | 0.71 | 1.12 |
| 7.3-3 | | 1 | 0.31 | 0.39 | 0.48 | 0.57 | 0.68 | 0.82 |
| 7.3-4 | | 2 | 0.33 | 0.37 | 0.43 | 0.49 | 0.62 | 0.79 |
| 7.3-5 | | 5 | 0.32 | 0.37 | 0.45 | 0.49 | 0.59 | 0.75 |
| 7.3-6 | 48 | 0 | 0.34 | 0.9 | 1.64 | 1.62 | 1.59 | 2.24 |
| 7.3-7 | | 0.5 | 0.32 | 0.59 | 1.16 | 1.36 | 1.43 | 2.01 |
| 7.3-8 | | 1 | 0.33 | 0.53 | 0.87 | 1.17 | 1.28 | 1.76 |
| 7.3-9 | | 2 | 0.31 | 0.47 | 0.73 | 0.93 | 1.04 | 1.62 |
| 7.3-10 | | 5 | 0.31 | 0.41 | 0.53 | 0.56 | 0.60 | 1.03 |

It can be seen from Table 42 that: during 55 days of accelerated storage at 37°C, in the formulations containing 3 mg/mL Compound 1 with different concentrations (1-5 mM) of citric acid monohydrate, the HMWP growth rates are essentially consistent, and all are superior to that of formulation 7.3-2 (containing 0.5 mM citric acid monohydrate). Formulation 7.3-2, containing 0.5 mM citric acid monohydrate, shows a significantly slower HMWP growth rate than that of the citric acid monohydrate-free formulation 7.3-1. In the formulations containing 48 mg/mL Compound 1, the HMWP growth rate decreases with increasing citric acid monohydrate concentration.

### Example 8: Stability Study of Compound 1 Formulations

### 8.1 Preparation and Filling of Formulations

Compound 1 formulations 8-1 to 8-9 were prepared and filled according to Table 43.

**Table 43: Formulations of compound 1**

| Formulation No. | Compound 1 (mg/mL) | NaCl (mg/mL) | Propylene glycol (mg/mL) | Citric acid monohydrate mM | Phenol (mg/mL) | Na₂HPO₄ (mg/mL) | pH | Devices |
|---|---|---|---|---|---|---|---|---|
| 8-1 | 3 | - | 14 | - | | | | Pre-filled syringe |
| 8-2 | 48 | | | | - | 1.42 | 7.3 | Pre-filled syringe |
| 8-3 | 3 | 7 | - | 2 | | | | Pre-filled syringe |
| 8-4 | 48 | | | | | | | Pre-filled syringe |

### 8.2 Detection of Related Substances

After the aforementioned formulations 8-1 to 8-4 were filled into their corresponding devices, stability studies were conducted at 4°C, 25°C, and 37°C. The increase in related substances over different days relative to Day 0 was measured for each formulation (formulations 8-1 to 8-4) in Example 8.1. The test results are shown in Tables 44, 45, and 46, respectively.

**Table 44: Related substances detecting results for compound 1 formulations under long-term storage conditions at 4°C**

| Formulation No. | Compound 1(mg/mL) | Day 0 | Day 27 | Day 53 |
|---|---|---|---|---|
| 8-1 | 3 | 2.29 | 2.53 | 2.55 |
| 8-2 | 48 | 2.19 | 3.08 | 2.49 |
| 8-3 | 3 | 2.25 | 3.06 | 2.55 |
| 8-4 | 48 | 2.19 | 2.64 | 2.49 |

It can be seen from Table 44 that: the change trends of related substances for each formulations were essentially consistent during long-term storage at 4°C. There was no significant difference in the stability of the various Compound 1 formulations during the 53-day storage period at 4°C.

**Table 45: Related substances detecting results for compound 1 formulations under accelerated conditions at 25°C**

| Formulation No. | Compound 1(mg/mL) | Day 0 | Day 27 | Day 53 |
|---|---|---|---|---|
| 8-1 | 3 | 2.29 | 8.85 | 9.27 |
| 8-2 | 48 | 2.19 | 3.95 | 4.94 |
| 8-3 | 3 | 2.25 | 3.42 | 3.54 |
| 8-4 | 48 | 2.19 | 2.88 | 3.55 |

It can be seen from Table 45 that: the stability of formulations 8-3 and 8-4 is significantly superior to that of formulations 8-1 and 8-2.

**Table 46: Detection results of related substances for compound 1 formulations under accelerated conditions at 37°C**

| Formulation No. | Compound 1 (mg/mL) | Day 0 | Day 5 | Day 12 | Day 20 | Day 27 | Day 53 |
|---|---|---|---|---|---|---|---|
| 8-1 | 3 | 2.29 | 3.05 | 6.03 | 9.28 | 14.66 | 25.57 |
| 8-2 | 48 | 2.19 | 3.23 | 4.28 | 5.41 | 6.88 | 8.47 |
| 8-3 | 3 | 2.25 | 2.85 | 3.50 | 3.85 | 5.32 | 7.66 |
| 8-4 | 48 | 2.19 | 2.90 | 3.56 | 4.23 | 6.13 | 9.04 |

It can be seen from Table 46 that: the stability of formulation 8-3 is significantly superior to that of formulation 8-1. The stability of formulation 8-4 is superior to or comparable to that of formulation 8-2.

### 8.3 HMWP Detection

Similar to Section 8.2, the increase in HMWP relative to Day 0 was measured at 4°C, 25°C, and 37°C for each formulation in Example 3. The test results are shown in Tables 47, 48, and 49, respectively.

**Table 47: HMWP detection results for compound 1 formulations under long-term 4°C conditions**

| Formulation No. | Compound 1(mg/mL) | Day 0 | Day 27 | Day 53 |
|---|---|---|---|---|
| 8-1 | 3 | 0.32 | 0.38 | 0.33 |
| 8-2 | 48 | 0.29 | 0.35 | 0.42 |
| 8-3 | 3 | 0.30 | 0.35 | 0.44 |
| 8-4 | 48 | 0.34 | 0.40 | 0.40 |

It can be seen from Table 47 that: the change trends in HMWP were essentially consistent across all groups during long-term storage at 4°C. There was basically no difference in the stability trends (changes in HMWP content) among the different Compound 1 formulations during the 53-day storage period at 4°C.

**Table 48: HMWP detection results for compound 1 formulations under accelerated conditions at 25°C**

| Formulation No. | Compound 1(mg/mL) | Day 0 | Day 27 | Day 53 |
|---|---|---|---|---|
| 8-1 | 3 | 0.32 | 0.74 | 1.08 |
| 8-2 | 48 | 0.29 | 1.02 | 1.65 |
| 8-3 | 3 | 0.30 | 0.39 | 0.50 |
| 8-4 | 48 | 0.34 | 0.50 | 0.57 |

It can be seen from Table 48 that: compared to formulations 8-1 and 8-2, formulations 8-3 and 8-4 demonstrate significantly superior stability.

**Table 49: HMWP results in comparative experiments for compound 1 formulations under accelerated conditions at 37°C**

| Formulation No. | Compound 1 (mg/mL) | Day 0 | Day 5 | Day 12 | Day 20 | Day 27 | Day 53 |
|---|---|---|---|---|---|---|---|
| 8-1 | 3 | 0.32 | 0.78 | 1.93 | 3.83 | 5.38 | 10.03 |
| 8-2 | 48 | 0.29 | 0.86 | 2.04 | 1.52 | 1.81 | 2.18 |
| 8-3 | 3 | 0.30 | 0.38 | 1.40 | 0.52 | 0.61 | 0.92 |
| 8-4 | 48 | 0.34 | 0.42 | 0.45 | 0.82 | 1.21 | 1.77 |

It can be seen from Table 49 that: the stability of formulations 8-3 and 8-4 is significantly superior to that of formulations 8-1 and 8-2.

### Sequences:

SEQ ID NO.1:
   GLP-1-(7-37) Peptide
   His Ala Glu Gly Thr Phe Thr Ser Asp Val Ser Ser Tyr Leu Glu Gly Gln Ala Ala Lys Glu Phe Ile Ala Trp Leu Val Lys Gly Arg Gly
SEQ ID NO.2:
   [Gly8, Arg34]GLP-1-(7-37) Peptide
   His Gly Glu Gly Thr Phe Thr Ser Asp Val Ser Ser Tyr Leu Glu Gly Gln Ala Ala Lys Glu Phe Ile Ala Trp Leu Val Arg Gly Arg Gly

The present invention has been described through the above examples, but it should be understood that the above examples are provided for illustrative purposes only and are not intended to limit the scope of the invention to the described examples. Furthermore, those skilled in the art will appreciate that the present invention is not limited to the above examples. Additional variations and modifications may be made based on the teachings of the present invention, and such variations and modifications fall within the scope of protection claimed herein. The scope of protection of the present invention is defined by the appended claims and their equivalents.

## Claims

1. A liquid pharmaceutical composition comprising:
a. a GLP-1 peptide or a pharmaceutically acceptable salt thereof;
b. an osmotic pressure regulator;
c. an antioxidant; and
d. a buffer;
wherein, the GLP-1 peptide is selected from the following compounds:
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Arg34]GLP-1-(7-37) peptide,
*N*-ε²⁶-(23-carboxytricosanoylamino)-4(*S*)-carboxybutanoyl-[Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
*N*-ε²⁶-(20-carboxyeicosanoylamino)-4(*S*)-carboxybutanoyl-[Arg34]GLP-1-(7-37) peptide, and
*N*-ε²⁶-(22-carboxydocosanoylamino)-4(*S*)-carboxybutanoyl-[Arg34]GLP-1-(7-37) peptide;
preferably, the GLP-1 peptide is selected from the following compounds:
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide, and
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide.
preferably, the GLP-1 peptide is the following compound: 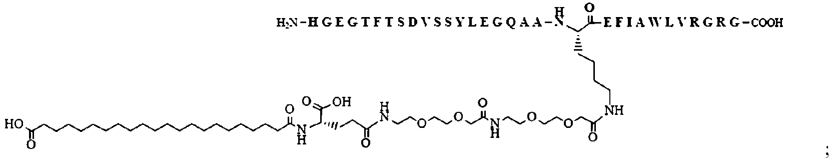
or

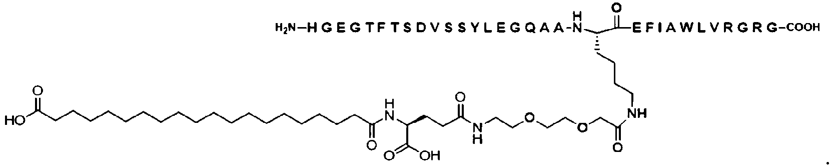

2. The pharmaceutical composition according to claim 1, wherein the pharmaceutical composition comprises or does not comprise a preservative, preferably the pharmaceutical composition does not comprise a preservative, preferably the preservative is phenol or m-cresol, preferably the pharmaceutical composition does not comprise phenol or m-cresol; and/or
the buffer is at least one selected from the group consisting of an acetic acid-sodium acetate buffer, acetic acid-potassium acetate buffer, acetic acid-ammonium acetate buffer, and a phosphate buffer, and preferably a phosphate buffer; preferably the phosphate buffer is Na₂HPO₄; and/or
the osmotic pressure regulator is NaCl or propylene glycol; and/or
the antioxidant is at least one selected from the group consisting of ascorbic acid, methionine, citric acid and tartaric acid; preferably, the antioxidant is selected from citric acid and methionine; preferably, the citric acid is citric acid monohydrate.

3. The pharmaceutical composition according to claim 2, **characterized in that** the pharmaceutical composition comprises more than about 0.5 mg/ml Na₂HPO₄, preferably about 0.5-20 mg/ml Na₂HPO₄, preferably about 1-20 mg/ml Na₂HPO₄, preferably about 1-10 mg/ml Na₂HPO₄, more preferably about 1-5 mg/ml Na₂HPO₄, and further preferably about 1.40 mg/ml, about 1.41 mg/ml, about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml Na₂HPO₄.

4. The pharmaceutical composition according to claim 2 or 3, wherein the concentration of NaCl is more than about 0.5 mg/ml, preferably more than about 1 mg/ml, preferably about 1-30 mg/ml, preferably about 3-25 mg/ml, preferably about 5-15 mg/ml, preferably about 5-10 mg/ml, preferably about 5 mg/ml, about 5.5 mg/ml, about 6 mg/ml, about 7 mg/ml, about 8 mg/ml, about 8.10 mg/ml, about 8.15 mg/ml, about 8.20 mg/ml, about 8.25 mg/ml, about 8.30 mg/ml, about 9 mg/ml or about 10 mg/ml; and/or
the concentration of propylene glycol is more than about 1 mg/ml, preferably more than about 2 mg/ml, preferably about 5-25 mg/ml, preferably about 10-20 mg/ml, preferably about 12-18 mg/ml, preferably about 13 mg/ml, about 13.5 mg/ml, about 14 mg/ml, about 14.5 mg/ml, about 15 mg/ml, about 16 mg/ml or about 17 mg/ml; and/or
the concentration of the antioxidant is more than about 0.5 mM, preferably more than about 1 mM, preferably about 1-25 mM, preferably about 1-20 mM, preferably about 1-15 mM, preferably about 1-10 mM, preferably about 1 mM, about 2 mM, about 3 mM, about 4 mM, about 5 mM, about 6 mM, about 7 mM, about 8 mM or about 9 mM.

5. The pharmaceutical composition according to any one of claims 2 to 4, wherein the antioxidant is selected from citric acid at a concentration of about 1-25 mM, preferably at a concentration of about 1-20 mM, preferably at a concentration of about 1-15 mM, preferably at a concentration of about 1-10 mM, preferably at a concentration of about 1 mM, about 2 mM, about 3 mM, about 4 mM, about 5 mM, about 6 mM, about 7 mM, about 8 mM or about 9 mM; preferably the citric acid is citric acid monohydrate; and/or
the concentration of the GLP-1 peptide is at least about 1 mg/ml, preferably at least about 2 mg/ml, preferably about 1-80 mg/ml, preferably about 1-70 mg/ml, preferably about 1-65 mg/ml, preferably about 1-60 mg/ml, preferably about 2-60 mg/ml, preferably about 2-50 mg/ml, more preferably about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml, about 28 mg/ml, about 29 mg/ml, about 30 mg/ml, about 31 mg/ml, about 32 mg/ml, about 33 mg/ml, about 34 mg/ml, about 35 mg/ml, about 36 mg/ml, about 37 mg/ml, about 38 mg/ml, about 39 mg/ml, about 40 mg/ml, about 41 mg/ml, about 42 mg/ml, about 43 mg/ml, about 44 mg/ml, about 45 mg/ml, about 46 mg/ml, about 47 mg/ml, about 48 mg/ml, about 49 mg/ml, or about 60 mg/ml, more preferably about 2 mg/ml, about 3 mg/ml, about 5 mg/ml, about 6 mg/ml, about 12 mg/ml, about 15 mg/ml, about 18 mg/ml, about 24 mg/ml, about 30 mg/ml, about 36 mg/ml, or about 48 mg/ml; further preferably about 3 mg/ml, about 6 mg/ml, about 12 mg/ml, about 18 mg/ml, about 24 mg/ml, about 36 mg/ml, or about 48 mg/ml; and/or
the pH of the pharmaceutical composition is about 6.5 to about 8.5, more preferably about 7.0 to about 8.5, further preferably about 7.1 to about 8.3, further preferably about 7.2, about 7.3, about 7.4, about 7.5, about 7.6, about 7.7, about 7.8, about 7.9, about 8.0, about 8.1, or about 8.2.

6. A liquid pharmaceutical composition comprising:
about 1-60 mg/ml, preferably about 1-70 mg/ml, preferably about 1-65 mg/ml, preferably about 1-60 mg/ml, preferably about 2-60 mg/ml, preferably about 2-50 mg/ml, more preferably about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml, about 28 mg/ml, about 29 mg/ml, about 30 mg/ml, about 31 mg/ml, about 32 mg/ml, about 33 mg/ml ml, about 34 mg/ml, about 35 mg/ml, about 36 mg/ml, about 37 mg/ml, about 38 mg/ml, about 39 mg/ml, about 40 mg/ml, about 41 mg/ml, about 42 mg/ml, about 43 mg/ml, about 44 mg/ml, about 45 mg/ml, about 46 mg/ml, about 47 mg/ml, about 48 mg/ml, about 49 mg/ml, further preferably about 2 mg/ml, about 3 mg/ml, about 5 mg/ml, about 6 mg/ml, about 12 mg/ml, about 15 mg/ml, about 18 mg/ml, about 24 mg/ml, about 30 mg/ml, about 36 mg/ml, or about 48 mg/ml; further preferably about 3 mg/ml, about 6 mg/ml, about 12 mg/ml, about 18 mg/ml, about 24 mg/ml, about 36 mg/ml, or about 48 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 1-30 mg/ml, preferably about 3-25 mg/ml, preferably about 5-15 mg/ml, preferably about 5 mg/ml, about 6 mg/ml, about 7 mg/ml, about 8 mg/ml, about 8.10 mg/ml, about 8.15 mg/ml, about 8.20 mg/ml, about 8.25 mg/ml, about 8.30 mg/ml, about 8.50 mg/ml, about 9 mg/ml or about 10 mg/ml of NaCl;
about 1-25 mM, preferably about 1-20 mM, more preferably about 1-15 mM, further preferably about 1-10 mM, further preferably about 1 mM, about 2 mM, about 3 mM, about 4 mM, about 5 mM, about 6 mM, about 7 mM, about 8 mM or about 9 mM citric acid;
about 1-20 mg/ml, preferably about 1-10 mg/ml, more preferably about 1-5 mg/ml, and further preferably about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of Na₂HPO₄; and
the pH of the pharmaceutical composition is about 6.5 to about 8.5, more preferably about 7.0 to about 8.5, further preferably about 7.1 to about 8.3, further preferably about 7.2, about 7.3, about 7.4, about 7.5, about 7.6, about 7.7, about 7.8, about 7.9, about 8.0, about 8.1, or about 8.2.

7. A liquid pharmaceutical composition comprising:
about 1-60 mg/ml, preferably about 1-70 mg/ml, preferably about 1-65 mg/ml, preferably about 1-60 mg/ml, preferably about 2-60 mg/ml, preferably about 2-50 mg/ml, more preferably about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13mg/ml, about 14mg/ml, about 15mg/ml, about 16mg/ml, about 17mg/ml, about 18mg/ml, about 19mg/ml, about 20mg/ml, about 21mg/ml, about 22mg/ml, about 23mg/ml, about 24mg/ml, about 25mg/ml, about 26mg/ml, about 27mg/ml, about 28mg/ml, about 29mg/ml, about 30mg/ml, about 31mg/ml, about 32 mg/ml, about 33mg/ml, about 34mg/ml, about 35mg/ml, about 36mg/ml, about 37mg/ml, about 38mg/ml, about 39mg/ml, about 40mg/ml, about 41mg/ml, about 42mg/ml, about 43mg/ml, about 44mg/ml, about 45mg/ml, about 46mg/ml, about 47mg/ml, about 48mg/ml, or about 49mg/ml, further preferably about 2 mg/ml, about 3 mg/ml, about 5 mg/ml, about 6 mg/ml, about 12 mg/ml, about 15 mg/ml, about 18 mg/ml, about 24 mg/ml, about 30 mg/ml, about 36 mg/ml, or about 48 mg/ml; further preferably about 3 mg/ml, about 6 mg/ml, about 12 mg/ml, about 18 mg/ml, about 24 mg/ml, about 36 mg/ml, or about 48 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 5-25 mg/ml, preferably about 10-20 mg/ml, preferably about 12-18 mg/ml, preferably about 13 mg/ml, about 13.5 mg/ml, about 14 mg/ml, about 14.5 mg/ml, about 15 mg/ml, 16 mg/ml or about 17 mg/ml of propylene glycol;
about 1-25 mM, preferably about 1-20 mM, more preferably about 1-15 mM, further preferably about 1-10 mM, further preferably about 1 mM, about 2 mM, about 3 mM, about 4 mM, about 5 mM, about 6 mM, about 7 mM, about 8 mM or about 9 mM citric acid;
about 1-20 mg/ml, preferably about 1-10 mg/ml, more preferably about 1-5 mg/ml, and further preferably about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of Na₂HPO₄; and
the pH of the pharmaceutical composition is about 6.5 to about 8.5, more preferably about 7.0 to about 8.5, further preferably about 7.1 to about 8.3, further preferably about 7.2, about 7.3, about 7.4, about 7.5, about 7.6, about 7.7, about 7.8, about 7.9, about 8.0, about 8.1, or about 8.2.

8. A liquid pharmaceutical composition comprising:
about 3 mg/ml, about 6 mg/ml, about 12 mg/ml, about 18 mg/ml, about 24 mg/ml, about 30 mg/ml, about 36 mg/ml, about 48 mg/ml or about 60 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N-*ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 7 mg/ml, about 8 mg/ml, about 8.10 mg/ml, about 8.15 mg/ml, about 8.20 mg/ml, about 8.25 mg/ml, about 8.30 mg/ml, about 8.50 mg/ml, about 9 mg/ml, or about 10 mg/ml of NaCl;
about 1 mM, about 2 mM, about 3 mM, about 4 mM, about 5 mM, about 6 mM, about 7 mM, about 8 mM, 9 mM or about 10 mM citric acid;
about 1.42 mg/ml of Na₂HPO₄; and
the pH of the pharmaceutical composition is about 7.3.

9. A liquid pharmaceutical composition comprising:
about 3 mg/ml, about 6 mg/ml, about 12 mg/ml, about 18 mg/ml, about 24 mg/ml, about 30 mg/ml, about 36 mg/ml, or about 48 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N-*ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 14 mg/ml of propylene glycol;
about 1 mM, 2 mM, about 3 mM, about 4 mM, about 5 mM, about 6 mM, about 7 mM, about 8 mM, 9 mM or about 10 mM citric acid;
about 1.42 mg/ml of Na₂HPO₄; and
the pH of the pharmaceutical composition is about 7.3.

10. A pharmaceutical product comprising a container and the liquid pharmaceutical composition according to any one of claims 1 to 9 placed in the container; preferably, the container is selected from a pen injection device, an automatic injection device, a syringe, and a vial.

11. A pharmaceutical product comprising a container and a liquid pharmaceutical composition placed in the container, **characterized in that** the container is deoxygenated and filled with nitrogen before the liquid pharmaceutical composition is placed in the container and/or during the process of the liquid pharmaceutical composition being placed in the container and/or after the liquid pharmaceutical composition is placed in the container; the liquid pharmaceutical composition comprises:
about 3 mg/ml, about 6 mg/ml, about 12 mg/ml, about 18 mg/ml, about 24 mg/ml, about 36 mg/ml, about 48 mg/ml, or about 60 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N-*ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 3 mg/ml, about 4 mg/ml, about 5 mg/ml, about 6 mg/ml, about 7 mg/ml, about 8 mg/ml, about 8.10 mg/ml, about 8.15 mg/ml, about 8.20 mg/ml, about 8.25 mg/ml, about 8.30 mg/ml, about 8.50 mg/ml, about 9 mg/ml, or about 10 mg/ml of NaCl;
about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of Na₂HPO₄; and
the pH of the pharmaceutical composition is about 7.0, about 7.1, about 7.2, about 7.3, about 7.4, about 7.6, or about 7.7.

12. A pharmaceutical product comprising a container and a liquid pharmaceutical composition placed in the container, **characterized in that** the container is deoxygenated and filled with nitrogen before the liquid pharmaceutical composition is placed in the container and/or during the process of the liquid pharmaceutical composition being placed in the container and/or after the liquid pharmaceutical composition is placed in the container; the liquid pharmaceutical composition comprises:
about 2-60 mg/ml, preferably about 3 mg/ml, about 6 mg/ml, about 12 mg/ml, about 18 mg/ml, about 24 mg/ml, about 36 mg/ml, or about 48 mg/ml of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide or *N-*ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide;
about 13 mg/ml, about 14 mg/ml, about 15 mg/ml, 16 mg/ml, or about 17 mg/ml of propylene glycol;
about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of Na₂HPO₄; and
the pH of the pharmaceutical composition is about 7.0, about 7.1, about 7.2, about 7.3, about 7.4, about 7.6, or about 7.7.

13. The pharmaceutical product according to claim 11 or 12, wherein the liquid pharmaceutical composition further comprises about 0.005 mM-25 mM, preferably about 0.01 mM-10 mM, further preferably about 0.1 mM-2.0 mM citric acid or methionine; and/or
deoxygenation of the container is achieved by filling the container with an inert gas; preferably, the inert gas is nitrogen; and/or
the container is a pen injection device, an automatic injection device, a syringe, or a vial.

14. The pharmaceutical composition according to any one of claims 1 to 9, or the pharmaceutical product according to any one of claims 10 to 13, wherein the pharmaceutical composition is for parenteral administration; preferably, the parenteral administration is subcutaneous administration.

15. A kit comprising a liquid pharmaceutical composition and instructions for use, wherein the liquid pharmaceutical composition is as defined in any one of claims 1 to 14.

16. A kit comprising a liquid pharmaceutical composition and an injection device, wherein the liquid pharmaceutical composition is as defined in any one of claims 1 to 14, and the injection device is for administering the composition to a subject, wherein the injection device is selected from a durable pen and a prefilled pen injection device.

17. Use of the pharmaceutical composition according to any one of claims 1 to 9, or the pharmaceutical product according to any one of claims 10 to 14, in the preparation of a medicament for treating diabetes, obesity, non-alcoholic fatty liver disease, Alzheimer's disease, or Parkinson's disease; preferably administering the pharmaceutical composition according to any one of claims 1 to 9 to a subject in need thereof once a week, once every two weeks, or less frequently.

18. The pharmaceutical composition according to any one of claims 1 to 9, or the pharmaceutical product according to any one of claims 10 to 14, for use in the treatment of diabetes, obesity, non-alcoholic fatty liver disease, Alzheimer's disease, or Parkinson's disease; preferably, the pharmaceutical composition according to any one of claims 1 to 9 is administered to a subject in need thereof once a week, once every two weeks, or less frequently.

19. A method for treating diabetes, obesity, non-alcoholic fatty liver disease, Alzheimer's disease, or Parkinson's disease, the method comprising administering the pharmaceutical composition according to any one of claims 1 to 19, or the pharmaceutical product according to any one of claims 10 to 14 to a subject in need thereof; preferably, administering the pharmaceutical composition according to any one of claims 1 to 9 to a subject in need thereof once a week, once every two weeks, or less frequently.

20. A method for preparing the pharmaceutical composition according to any one of claims 1 to 9, **characterized in that** the method comprises:
(1) dissolving a formulated amount of a buffer, an osmotic pressure regulator, and/or an antioxidant in water;
(2) dissolving a formulated amount of a GLP-1 peptide in the solution obtained in step (1), and adjusting the pH using sodium hydroxide and/or dilute hydrochloric acid;
(3) finally sterilizing the solution obtained in step (2) by filtering through a 0.22 µm sterile filter.

21. A method for preparing the pharmaceutical composition according to any one of claims 1 to 9, wherein the method comprises:
mixing the GLP-1 peptide, buffer, osmotic pressure regulator and antioxidant according to the formulation;
preferably, the method further comprises:
sterilizing the mixture obtained in step (2); preferably, the mixture obtained in step (2) is sterilized by filtering through a 0.22 µm sterile filter.
